# EUROPÄISCHE PATENTANMELDUNG

(11) **EP 0 542 681 A1**
(43) Veröffentlichungstag der Anmeldung: **19.05.1993**
(21) Anmeldenummer: 92810847.1
(22) Anmeldetag: 03.11.1992
(51) Int. Cl.: C07D 473/00, C07D 487/04, C07D 471/04, C07D 239/46, C07D 239/54, C07D 327/10, C07D 319/08, C07C 35/06, C07C 43/178

(54) **Verfahren zur Herstellung von carbacyclischen Nukleosiden und Zwischenprodukte**

(30) Priorität: 12.11.1991 CH 3292/91
(71) Anmelder: CIBA-GEIGY AG, CH-4002 Basel (CH)
(72) Erfinder: Moser, Heinz, Dr., CH-4313 Möhlin (CH)

(57) **Zusammenfassung**

Verfahren zur Herstellung von racemischen Verbindungen der Formel I 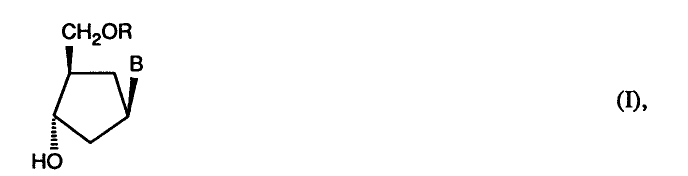
oder deren Enantiomeren, worin B für den Rest einer Nukleinbase aus der Reihe Purin, einem Pyrinanalogen oder einem Pyridinanalogen steht und R H oder eine Schutzgruppe R₁ bedeutet, durch Umsetzung
a) einer Verbindung der Formel II oder deren Enantiomeren 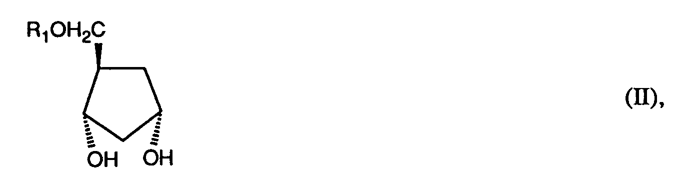 mit einer Verbindung der Formeln X-CO-X, X-CS-X, X-SO-X, X-SO₂-X, R₂-NCO, R₃R₄N-C(O)-R₅, X₂P-R₆ oder X₂P(O)-R₆, worin X eine Abgangsgruppe bedeutet, R₂ zum Beispiel für C₁-C₁₈-Alkyl steht, R₃ und R₄ zum Beispiel H oder C₁-C₁₈-Alkyl bedeuten, R₅ zum Beispiel für H oder C₁-C₁₂-Alkyl steht, und R₆ zum Beispiel C₁-C₁₂-Alkyl oder -Alkoxy bedeutet, zu einer Verbindung der Formel III oder deren Enantiomeren, 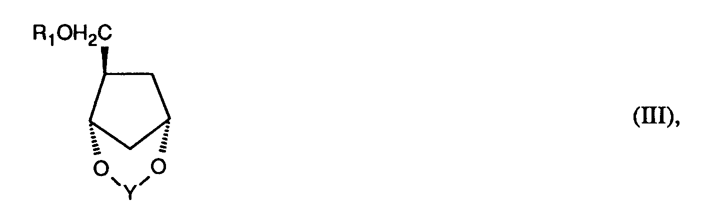 worin Y die Gruppen -C(O)-, -C(S)-, -SO-, -SO₂-, R₂NH-CH=, R₃R₄N-CR₅=, R₆P= und R₆(O)P= darstellt;
b) die weitere Umsetzung der Verbindungen der Formeln Ill oder deren Enantiomeren mit einer Nukleinbase B alleine oder zusammen mit einer nicht-nukleophilen Base, oder mit einem Alkalimetallsalz der Nukleinbase B zu Verbindungen der Formel IV oder deren Enantiomeren 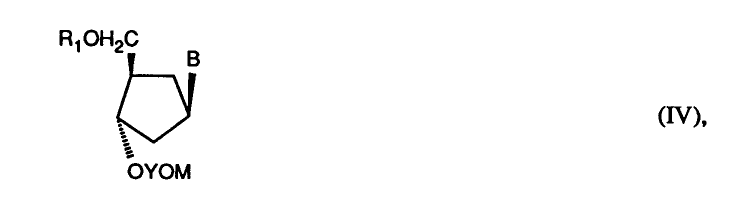 worin M für H, ein Alkalimetall oder eine nicht-nukleophile Base steht,und
c) die Umwandlung der Verbindungen der Formeln IV durch Abspaltung der Schutzgruppe R₁ und der Gruppe YOM in die Verbindungen der Formeln I. Die Umsetzung b) ist regioselektiv und diastereoselektiv und man erhält die Verbindungen der Formel I in hohen Ausbeuten und Reinheiten.

## Beschreibung

Die Erfindung betrifft ein Verfahren zur Herstellung von carbacyclischen Nukleosiden durch die Umsetzung von geschützten und überbrückten Transhydroxymethyl-cis-1,3-pentandiolen mit einer Nukleinbase aus der Reihe Purin, Purinanaloge und Pyrimidinanaloge, und einer anschliessenden Abspaltung der Brücken- und Schutzgruppe.

Carbacyclische Nukleosidanaloge haben auf Grund Ihrer antiviralen Eigenschaften und als Bausteine für Oligonukleotide mit Antisense-Eigenschaften Interesse erlangt, siehe zum Beispiel M. Bodenteich et al., Nucleosides & Nucleotides, 6(182), Seiten 233-237 1987, A. Szemzo et al., Tetrahedron Letters, Vol. 31, No. 10, Seiten 1463-1466 (1990), K. Biggadike et al., J. Chem. Soc., Chem. Commun., Seiten 1083-1084 (1987), J. Balzarini et al., J. Med. Chem., Vol. 32, Seiten 1861-1865 (1989), J. Beres et al., J. Med. Chem., Vol. 33, Seiten 1353-1360 (1990) und V. E. Marquez et al., Med. Res. Rev., Vol. 6, Seiten 1-40 (1986). Die bekannten Synthesemethoden sind sehr aufwendige Mehrstufenverfahren, mit denen die gewünschten Nukleosidanalogen in nur geringen Gesamtausbeuten erhalten werden. Für die Synthese der carbacyclischen, sowohl racemischer als auch enantiomerenreiner Nukleosidanalogen in hohen Ausbeuten ist noch kein befriedigendes Verfahren gefunden worden. Ein Verfahren zur gleichzeitigen Herstellung von solchen Nukleosidanalogen der natürlichen und unnatürlichen Reihe unter Verwendung eines Ausgangsproduktes ist ebenfalls nicht bekannt.

Es wurde nun gefunden, dass man solche Nukleosidanaloge in hohen Ausbeuten und Reinheiten sowohl in racemischer als auch enantioselektiver Form herstellen kann, wenn man von den nach einem neuen Verfahren aus Cis-1,3-Pentendiol leicht zugänglichen geschützten Trans-4-Hydroxymethyl-cis-1,3-pentandiolen in Form der Racemate oder deren Enantiomeren ausgeht, die 1,3-Hydroxygruppen mit zum Beispiel SO₂-Gruppen überbrückt, diese Verbindungen mit einer Nukleinbase wie zum Beispiel Adenin umsetzt, und dann die Brücken- und Schutzgruppen in an sich bekannter Weise entfernt. Mit diesem Mehrstufenverfahren kann man gleichzeitig Nukleosidanaloge der natürlichen und unnatürlichen Reihe und die Racemate erhalten, wobei man trotz mehrerer Syntheseschritte hohe Gesamtausbeuten erzielt. Das Verfahren eignet sich daher auch für eine industrielle Produktion.

Ein Gegenstand der Erfindung ist ein Verfahren zur Herstellung von racemischen Verbindungen der Formel I 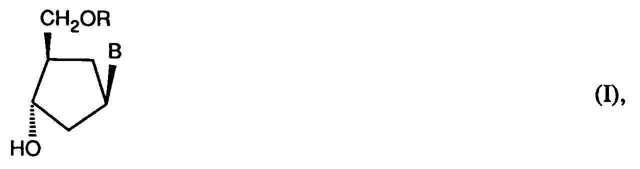
oder der Enantiomeren der Formeln (Ia) und (Ib) 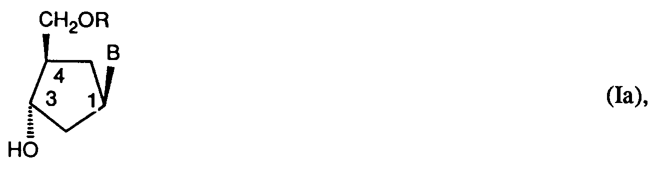 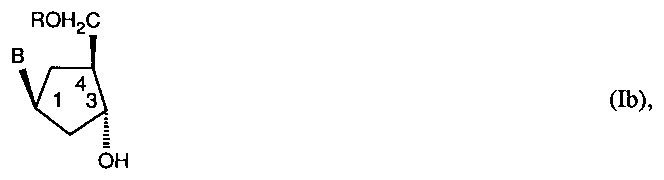
worin B für den Rest einer Nukleinbase aus der Reihe Purin oder einem Purinanalogen oder Pyrimidinanalogen steht und R H oder eine Schutzgruppe R₁ bedeutet, dadurch gekennzeichnet, dass man
a) eine Verbindung der Formel II in Form des Racemats oder deren Enantiomeren der Formeln IIa oder IIb 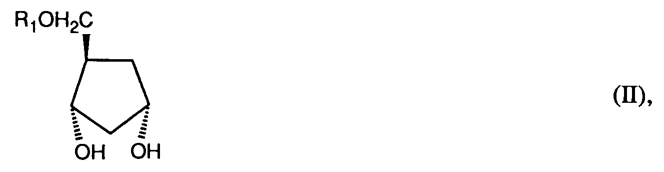 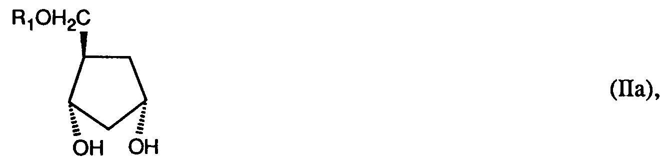 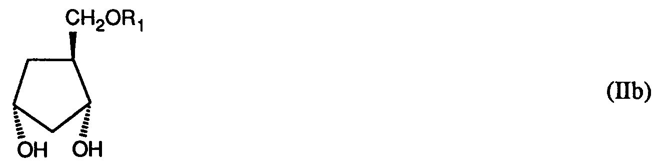 worin R₁ für eine Schutzgruppe steht, mit einer Verbindung der Formeln X-CO-X, X-CS-X, X-SO-X, X-SO₂-X, R₂-NCO, R₃R₄N-C(O)-R₅, X₂P-R₆ oder X₂P(O)-R₆, worin X eine Abgangsgruppe bedeutet, R₂ für lineares oder verzweigtes C₁-C₁₈-Alkyl, C₅-C₈-Cycloalkyl, Phenyl, Benzyl, oder mit C₁-C₆-Alkyl oder C₁-C₆-Alkoxy substituiertes Phenyl oder Benzyl steht, R₃ und R₄ unabhängig voneinander H, lineares oder verzweigtes C₁-C₁₈-Alkyl, C₅-C₈-Cycloalkyl, Phenyl, Benzyl, oder mit C₁-C₆-Alkyl oder C₁-C₆-Alkoxy substituiertes Phenyl oder Benzyl, oder R₃ und R₄ zusammen Tetramethylen, Pentamethylen oder 3-oxa-1,5-pentylen bedeuten, R₅ für H, lineares oder verzweigtes C₁-C₁₂-Alkyl, C₅-C₈-Cycloalkyl, Phenyl, Benzyl, oder mit C₁-C₆-Alkyl oder C₁-C₆-Alkoxy substituiertes Phenyl oder Benzyl steht, und R₆ lineares oder verzweigtes C₁-C₁₂-Alkyl oder -Alkoxy, C₅-C₈-Cycloalkyl oder -Cyclalkoxy, Phenyl oder Phenyloxy, Benzyl oder Benzyloxy, oder mit C₁-C₆-Alkyl oder C₁-C₆-Alkoxy substituiertes Phenyl oder Phenyloxy oder Benzyl oder Benzyloxy darstellt, zu Racematen der Formel III oder Enantiomeren der Formeln IIIa oder IIIb umsetzt, 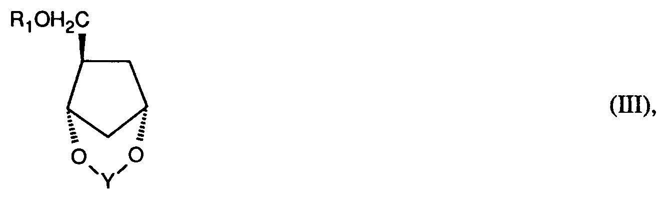 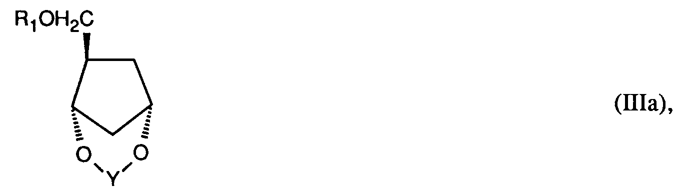 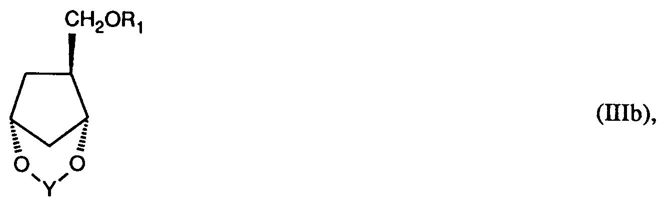 worin Y die Gruppen -C(O)-, -C(S)-, -SO-, -SO₂-, R₂NH-CH=, R₃R₄N-CR₅=, R₆P= und R₆(O)P= darstellt, worin R₁ und R₂ bis R₆ die zuvor angegebenen Bedeutungen haben,
b) die Verbindungen der Formeln III, IIIa oder IIIb mit einer Nukleinbase B, gegebenenfalls zusammen mit einer nicht-nukleophilen Base, oder Alkalimetallsalzen einer Nukleinbase B zu Racematen der Formeln IV oder zu Enantiomeren der Formeln IVa oder IVb 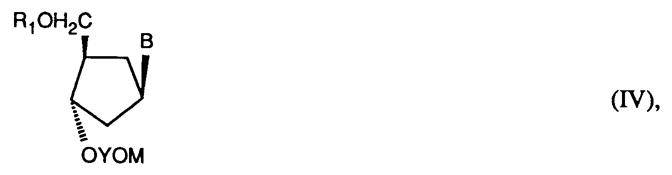 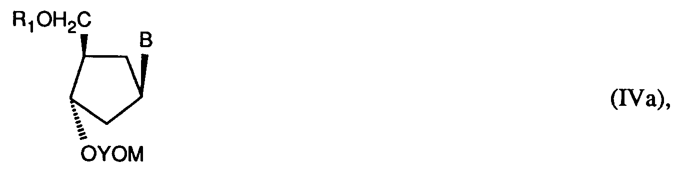 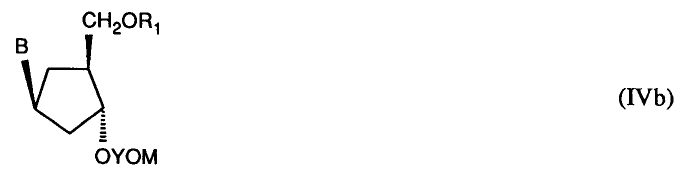 umsetzt, worin M für H, ein Alkalimetall oder eine nicht-nukleophile Base steht und Y die zuvor angegebene Bedeutung hat, und
c) die Verbindungen der Formeln IV, IVa und IVb durch Abspaltung der Schutzgruppe R₁ und der Gruppe YOM in die Verbindungen der Formeln I, Ia und Ib umwandelt.

Schutzgruppen und Verfahren zur Derivatisierung der Hydroxylgruppen mit solchen Schutzgruppen sind in der Zuckerchemie allgemein bekannt. Beispiele für solche Schutzgruppen sind: lineares oder verzweigtes C₁-C₈-, besonders C₁-C₄-Alkyl, zum Beispiel Methyl, Ethyl, n- und i-Propyl, n-, i- und t-Butyl; C₇-C₁₂-Aralkyl, zum Beispiel Benzyl, Methylbenzyl, Dimethylbenzyl, Methoxybenzyl, Dimethoxybenzyl, Brombenzyl; Diphenylmethyl, Di(methylphenyl)methyl, Di(dimethylphenyl)methyl, Di(methoxyphenyl)methyl, Di(dimethoxyphenyl)methyl, Trityl, Tri(methylphenyl)methyl, Tri(dimethylphenyl)methyl, Tri(methoxyphenyl)methyl, Tri(dimethoxyphenyl)methyl, Monomethoxytrityl, Dimethoxytrityl; Pixyl; Triphenylsilyl, Alkyldiphenylsilyl, Dialkylphenylsilyl und Trialkylsilyl mit 1 bis 20, bevorzugt 1 bis 12 und besonders bevorzugt 1 bis 8 C-Atomen in den Alkylgruppen, zum Beispiel Trimethylsilyl, Triethylsilyl, Tri-n-propylsilyl, i-Propyl-dimethylsilyl, t-Butyl-dimethylsilyl, t-Butyl-diphenylsilyl, n-Octyl-dimethylsilyl, (1,1,2,2-Tetramethylethyl)-dimethylsilyl; C₂-C₁₂-, besonders C₂-C₈-Acyl, wie zum Beispiel Acetyl, Propanoyl, Butanoyl, Pentanoyl, Hexanoyl, Benzoyl, Methylbenzoyl, Methoxybenzoyl, Chlorbenzoyl und Brombenzoyl; R₁₇-SO₂-, worin R₁₇ C₁-C₁₂-Alkyl, besonders C₁-C₆-Alkyl, C₅- oder C₆-Cycloalkyl, Phenyl, Benzyl, C₁-C₁₂- und besonders C₁-C₄-Alkylphenyl, oder C₁-C₁₂- und besonders C₁-C₄-Alkylbenzyl, oder Halogenphenyl oder Halogenbenzyl bedeutet, zum Beispiel Methyl-, Ethyl-, Propyl-, Butyl-, Phenyl-, Benzyl-, p-Brom-, p-Methoxy- und p-Methylphenylsulfonyl; C₁-C₁₂-, bevorzugt C₁-C₈-Alkoxycarbonyl, zum Beispiel Methoxy-, Ethoxy-, n- oder i-Propoxy- oder n-, i- oder t-Butoxycarbonyl, oder Phenyloxycarbonyl, Benzyloxycarbonyl, Methyl- oder Methoxy- oder Chlorphenyloxycarbonyl oder -benzyloxycarbonyl.

In einer bevorzugten Ausführungsform sind die Verbindungen der Formel I solche, worin R₁ unabhängig voneinander lineares oder verzweigtes C₁-C₄-Alkyl, C₇-C₁₂-Aralkyl, Trialkylsilyl mit 1 bis 12 C-Atomen in den Alkylgruppen, C₂-C₈-Acyl, R₁₇-SO₂-, worin R₁₇ C₁-C₆-Alkyl, Phenyl, Benzyl, C₁-C₄-Alkylphenyl, C₁-C₄-Alkylbenzyl, oder Halogenphenyl oder Halogenbenzyl bedeutet, oder C₁-C₈-Alkoxycarbonyl, Phenoxycarbonyl oder Benzyloxycarbonyl darstellen.

In einer besonders bevorzugten Ausführungsform stellt R₁ Methyl, Ethyl, n- und i-Propyl, n-, i- und t-Butyl; Benzyl, Methylbenzyl, Dimethylbenzyl, Methoxybenzyl, Dimethoxybenzyl, Brombenzyl; Diphenylmethyl, Di(methylphenyl)methyl, Di(dimethylphenyl)methyl, Di(methoxyphenyl)methyl, Di(dimethoxyphenyl)methyl, Trityl, Tri(methylphenyl)methyl, Tri(dimethylphenyl)methyl, Tri(methoxyphenyl)methyl, Tri(dimethoxyphenyl)methyl, Monomethoxytrityl, Dimethoxytrityl, Pixyl; Trimethylsilyl, Triethylsilyl, Tri-n-propylsilyl, i-Propyl-dimethylsilyl, t-Butyl-dimethylsilyl, t-Butyl-diphenylsilyl, n-Octyl-dimethylsilyl, (1,1,2,2-Tetramethylethyl)-dimethylsilyl; Acetyl, Propanoyl, Butanoyl, Pentanoyl, Hexanoyl, Benzoyl, Methylbenzoyl, Methoxybenzoyl, Chlorbenzoyl und Brombenzoyl; Methyl-, Ethyl-, Propyl-, Butyl-, Phenyl-, Benzyl-, p-Brom-, p-Methoxy- und p-Methylphenylsulfonyl; Methoxy-, Ethoxy-, n- oder i-Propoxy- oder n-, i- oder t-Butoxycarbonyl, oder Phenyloxycarbonyl, Benzyloxycarbonyl, Methyl- oder Methoxy- oder Chlorphenyloxycarbonyl oder -benzyloxycarbonyl dar.

Wenn B einen Purinrest oder ein Analoges davon darstellt, so kann es sich um Reste der Formeln V, Va, Vb, Vc, Vd oder Ve handeln, 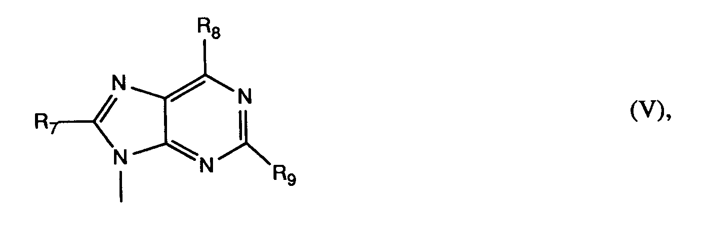 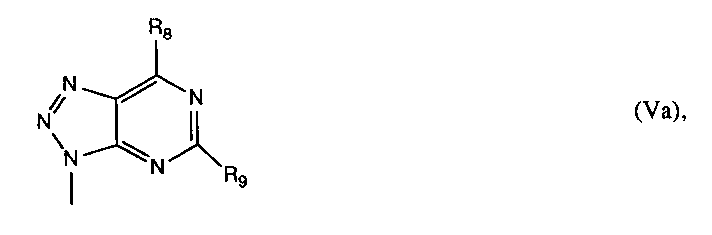 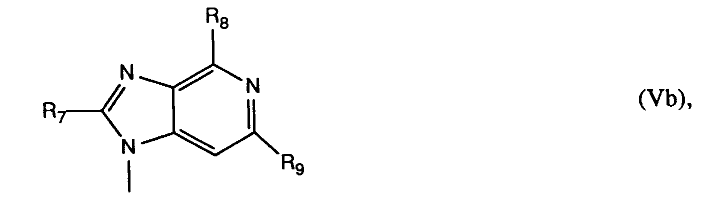 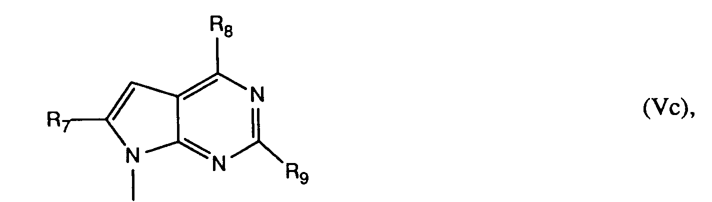 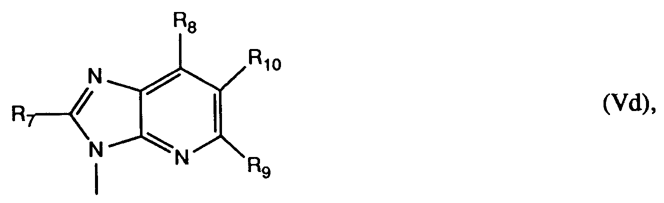 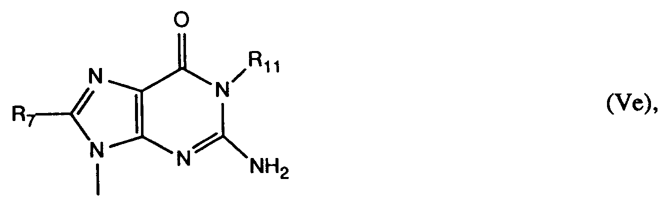
worin R₇ für H, Cl, Br, NH₂ oder OH steht, und R₈, R₉ und R₁₀ unabhängig voneinander H, OH, SH, NH₂, NHNH₂, NHOH, NHOAlkyl mit 1 bis 12 C-Atomen, F, Cl, Br, Alkyl oder Hydroxyalkyl oder Aminoalkyl oder Alkoxy oder Alkylthio mit 1 bis 12 C-Atomen, wobei die Hydroxyl- und Aminogruppen unsubstituiert oder mit einer Schutzgruppe substituiert sind, Phenyl, Benzyl, Primäramino mit 1 bis 20 C-Atomen oder Sekundäramino mit 2 bis 30 C-Atomen bedeuten, und R₁₁ H oder C₁-C₄-Alkyl darstellt.

Geeignete Schutzgruppen sind zuvor erwähnt worden. Bevorzugte Schutzgruppen sind C₁-C₈-Acylgruppen, wie zum Beispiel Acetyl, Propionyl, Butyroyl und Benzoyl. R₁₁ steht bevorzugt für H oder Methyl.

Das Primäramino enthält bevorzugt 1 bis 12 und besonders bevorzugt 1 bis 6 C-Atome, und das Sekundäramino bevorzugt 2 bis 12 und besonders bevorzugt 2 bis 6 C-Atome.

Einige Beispiele für Alkyl, Alkoxy, Alkylthio, Hydroxyalkyl und Aminoalkyl, die bevorzugt 1 bis 6 C-Atome enthalten, sind Methyl, Ethyl und die Isomeren von Propyl, Butyl, Pentyl, Hexyl, Heptyl, Octyl, Nonyl, Decyl, Undecyl und Dodecyl, sowie entsprechende Alkoxy-, Alkylthio-, Hydroxyalkyl- und Aminoalkylreste. Das Alkyl, Alkoxy, Alkylthio, Hydroxyalkyl und Aminoalkyl enthält besonders bevorzugt 1 bis 4 C-Atome. Bevorzugte Alkyl-, Alkoxy-, Alkylthio-, Hydroxyalkyl- und Aminolkylreste sind Methyl, Ethyl, n- und i-Propyl, n-, i- und t-Butyl, Methoxy, Ethoxy, Methylthio und Ethylthio, Aminomethyl, Aminoethyl, Hydroxymethyl und Hydroxyethyl.

Bei dem Primäramino und Sekundäramino kann es sich zum Beispiel um Reste der Formel R₁₄R₁₅N handeln, worin R₁₄ für H steht oder unabhängig die Bedeutung von R₁₅ hat, und R₁₅ C₁-C₂₀-, bevorzugt C₁-C₁₂- und besonders bevorzugt C₁-C₆-Alkyl, -Aminoalkyl, -Hydroxyalkyl; Carboxyalkyl oder Carbalkoxyalkyl, wobei die Carbalkoxygruppe 2 bis 8 C-Atome enthält und die Alkylgruppe 1 bis 6, bevorzugt 1 bis 4 C-Atome; C₂-C₂₀-, bevorzugt C₂-C₁₂- und besonders bevorzugt C₂-C₆-Alkenyl; Phenyl, Mono- oder Di-(C₁-C₄-Alkyl- oder -Alkoxy)phenyl, Benzyl, Mono- oder Di-(C₁-C₄-Alkyl- oder -Alkoxy)benzyl; oder 1,2-, 1,3- oder 1,4-Imidazolyl-C₁-C₆-Alkyl darstellt, oder R₁₄ und R₁₅ zusammen Tetra- oder Pentamethylen, 3-oxa- 1,5-pentylen, -CH₂-NR₁₆-CH₂CH₂- oder -CH₂CH₂-NR₁₆-CH₂CH₂- darstellen, worin R₁₆ für H oder C₁-C₄-Alkyl steht. Die Aminogruppe im Aminoalkyl kann mit ein oder zwei C₁-C₄-Alkyl oder -Hydroxyalkylgruppen substituiert sein. Die Hydroxylgruppe im Hydroxyalkyl kann mit C₁-C₄-Alkyl verethert sein.

Beispiele für Alkyl sind zuvor angegeben worden. Beispiele für Aminoalkyl sind Aminomethyl, Aminoethyl, 1-Aminoprop-2-yl oder -3-yl, 1-Amino-but-2-yl oder -3-yl oder -4-yl, N-Methyl- oder N,N-Dimethyl- oder N-Ethyl- oder N,N-Diethyl- oder N-2-Hydroxyethyl- oder N,N-Di-2-hydroxyethylaminomethyl oder -aminoethyl oder -aminopropyl oder -aminobutyl. Beispiele für Hydroxyalkyl sind Hydroxymethyl, 1-Hydroxy-eth-2-yl, 1-Hydroxy-prop-2- oder -3-yl, 1-Hydroxy-but-2-yl, -3-yl oder -4-yl. Beispiele für Carboxyalkyl sind Carboxymethyl, Carboxyethyl, Carboxypropyl und Carboxybutyl, und Beispiele für Carbalboxyalkyl sind diese mit Methyl oder Ethyl veresterten Carboxyalkylgruppen. Beispiele für Alkenyl sind Allyl, But-1-en-3-yl oder -4-yl, Pent-3- oder 4-en-1-yl oder -2-yl, Hex-3- oder -4- oder -5-en-1-yl oder -2-yl. Beispiele für Alkyl- und Alkoxyphenyl beziehungsweise -benzyl sind Methylphenyl, Dimethylphenyl, Ethylphenyl, Diethylphenyl, Methylbenzyl, Dimethylbenzyl, Ethylbenzyl, Diethylbenzyl, Methoxyphenyl, Dimethoxyphenyl, Ethoxyphenyl, Diethoxyphenyl, Methoxybenzyl, Dimethoxybenzyl, Ethoxybenzyl, Diethoxybenzyl. Beispiele für Imidazolylalkyl, in dem die Alkylgruppe bevorzugt 2 bis 4 C-Atome enthält, sind 1,2-, 1,3- oder 1,4-Imidazolylethyl oder -n-propyl oder -n-butyl. R₁₆ stellt bevorzugt H, Methyl oder Ethyl dar.

Bevorzugte Beispiele für Primäramino und Sekundäramino sind Methyl-, Ethyl-, Dimethyl-, Diethyl-, Allyl-, Mono- oder Di-(1-hydroxy-eth-2-yl)-, Phenyl- und Benzylamino, Acetylamino und Benzoylamino.

In einer bevorzugten Ausführungsform stellt R₇ Wasserstoff dar. In einer anderen bevorzugten Ausführungsform stellt R₁₀ Wasserstoff dar. In einer weiteren bevorzugten Ausführungsform bedeuten R₈ und R₉ unabhängig voneinander H, F, Cl, Br, OH, SH, NH₂, NHOH, NHNH₂, Methylamino, Dimethylamino, Benzoylamino, Methoxy, Ethoxy und Methylthio.

Einige Beispiele für Analoge der Purinreihe sind neben Purin Adenin, N-Methyladenin, N-Benzoyladenin, 2-Methylthioadenin, 2-Aminoadenin, 6-Hydroxypurin, 2-Amino-6-chlorpurin, 2-Amino-6-methylthiopurin, Guanin, N-Isobutyrylguanin. Besonders bevorzugt sind Adenin, 2-Aminoadenin und Guanin.

Wenn B in Formel I einen analogen Pyrimidinrest darstellt, so handelt es sich bevorzugt um Uracil-, Thymin- und Cytosinreste der Formeln VI, VIa und VIb, 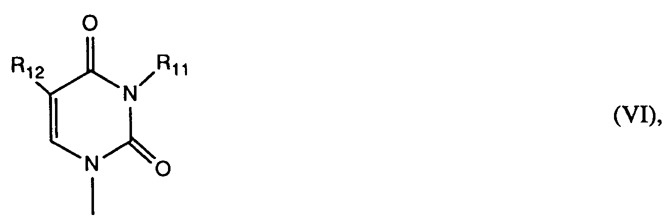 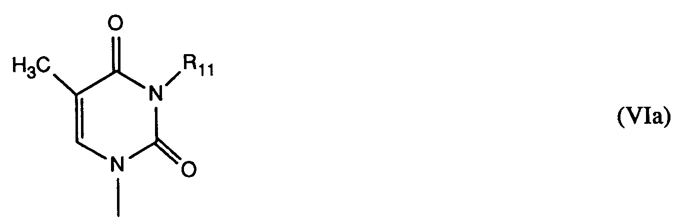 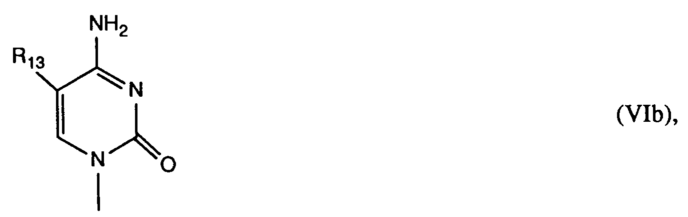
worin R₁₁ H oder C₁-C₄-Alkyl bedeutet, und R₁₂ und R₁₃ unabhängig voneinender die zuvor für R₈ angegebene Bedeutung haben, einschliesslich der Bevorzugungen, und die Wasserstoffatome der NH₂-Gruppe in Formel VIb mit C₁-C₆-Alkyl oder Benzoyl substituiert sein können, sowie die Dihydroderivate der Reste der Formeln VI, VIa und VIb. Bevorzugt stellt R₁₂ H, C₁-C₆-Alkyl oder Hydroxyalkyl, F, Cl, Br, NH₂, Benzoylamino, Mono- oder Di-C₁-C₆-alkylamino dar und R₁₃ stellt bevorzugt H, C₁-C₆-Alkyl oder -Alkoxy oder -Hydroxyalkyl, F, Cl, Br, NH₂, Benzoylamino, Mono- oder Di-C₁-C₆-alkylamino dar.

R₁₁ steht bevorzugt für H oder Methyl. R₁₂ bedeutet bevorzugt H, F, Cl, Br, NH₂, NHCH₃, N(CH₃)₂ oder C₁-C₄-Alkyl. R₁₃ stellt bevorzugt H, C₁-C₄-Alkyl, besonders Methyl, oder NH₂, NHCH₃ oder (CH₃)₂N dar.

Einige Beispiele Pyrimidinanaloge sind Uracil, Thymin, Cytosin, 5-Fluoruracil, 5-Chloruracil, 5-Bromuracil, Dihydrouracil und 5-Methylcytosin.

R₂ stellt bevorzugt C₁-C₁₂-, besonders bevorzugt C₁-C₆-Alkyl, C₅- oder C₆-Cycloalkyl, Phenyl, Benzyl, oder mit C₁-C₄-Alkyl oder C₁-C₄-Alkoxy substituiertes Phenyl oder Benzyl dar. Einige Beispiele für R₂ sind Methyl, Ethyl, n- oder i-Propyl, n- oder i-Butyl, Cyclopentyl, Cyclohexyl, Phenyl, Methylphenyl, Ethylphenyl, i-Propylphenyl, n- oder t-Butylphenyl, Dimethylphenyl, Methoxyphenyl und Dimethoxyphenyl.

R₃ und R₄ bedeuten bevorzugt H, C₁-C₁₂-, besonders bevorzugt C₁-C₆-Alkyl, C₅- oder C₆-Cycloalkyl, Phenyl, Benzyl, oder mit C₁-C₄-Alkyl oder C₁-C₄-Alkoxy substituiertes Phenyl oder Benzyl. Einige Beispiele für R₂ sind Methyl, Ethyl, n- oder i-Propyl, n- oder i-Butyl, Cyclopentyl, Cyclohexyl, Phenyl, Methylphenyl, Ethylphenyl, i-Propylphenyl, n- oder t-Butylphenyl, Dimethylphenyl, Methoxyphenyl und Dimethoxyphenyl.

R₅ stellt bevorzugt C₁-C₆-Alkyl, C₅- oder C₆-Cycloalkyl, Phenyl, Benzyl, oder mit C₁-C₄-Alkyl oder C₁-C₄-Alkoxy subsrituiertes Phenyl oder Benzyl dar. Einige Beispiele für R₂ sind Methyl, Ethyl, n- oder i-Propyl, n- oder i-Butyl, Cyclopentyl, Cyclohexyl, Phenyl, Methylphenyl, Ethylphenyl, i-Propylphenyl, n- oder t-Butylphenyl, Dimethylphenyl, Methoxyphenyl und Dimethoxyphenyl.

R₆ stellt bevorzugt C₁-C₆-Alkyl oder -Alkoxy, C₅- oder C₆-Cycloalkyl oder -Cycloalkoxy, Phenyl oder Phenyloxy, Benzyl oder Benzyloxy, oder mit C₁-C₄-Alkyl oder C₁-C₄-Alkoxy substituiertes Phenyl, Phenyloxy, Benzyl oder Benzyloxy dar. Einige Beispiele für R₆ sind Methyl, Ethyl, n- oder i-Propyl, n- oder i-Butyl, Methoxy, Ethoxy, Propyloxy, Butyloxy, Cyclopentyl, Cyclohexyl, Phenyl, Phenoxy, Methylphenyl, Ethylphenyl, i-Propylphenyl, n- oder t-Butylphenyl, Dimethylphenyl, Methoxyphenyl und Dimethoxyphenyl, Methylphenyloxy, Ethylphenyloxy, i-Propylphenyloxy, n- oder t-Butylphenyloxy, Dimethylphenyloxy, Methoxyphenyloxy und Dimethoxyphenyloxy, Benzyl, Benzyloxy, Methylbenzyl, Methoxybenzyl, Dimethylbenzyl und Dimethoxybenzyl.

Bei der Abgangsgruppe X kann es sich um Halogenid, bevorzugt Chlorid und Bromid, um C₁-C₆-, bevorzugt C₁-C₄-Alkoxy, Phenoxy, Benzyloxy oder um offenkettiges oder cyclisches Sekundäramino mit 2 bis 12 C-Atomen handeln. Beispiele für Sekundäramino sind Dimethylamino, Diethylamino, Di-n-butylamino, Diphenylamino, Dibenzylamino, Piperazinyl, Morpholinyl oder Imidazolyl. Bevorzugte Abgangsgruppen sind Chlorid, Bromid, Methoxy, Ethoxy und Imidazolyl. Die Umsetzung der Verfahrensstufe a) wird bevorzugt mit Verbindungen der Formel X-CO-X vorgenommen, worin X für Imidazolyl steht, oder mit einer Verbindung der Formel X-SO-X durchgeführt, worin X für Cl oder Br steht, wobei die entstehenden cyclischen Sulfoxidverbindungen anschliessend in bekannter Weise zu den entsprechenden cyclischen Sulfodioxidverbindungen oxidiert werden können.

Ein weiterer Gegenstand der Erfindung sind die Verbindungen der Formeln II, IIa und IIb , 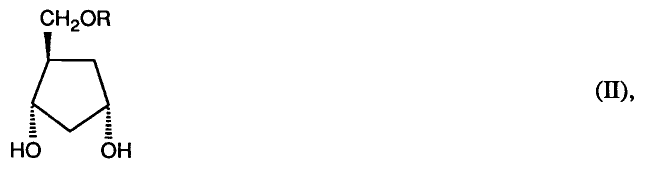
oder der Enantiomeren der Formeln (Ia) und (Ib) 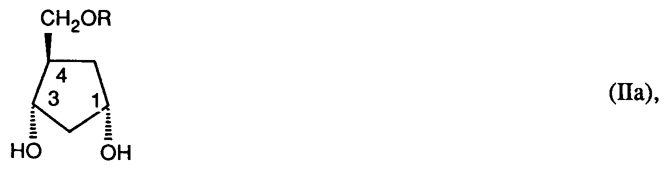 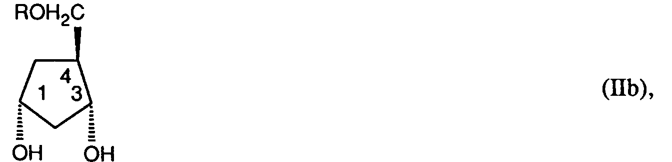
worin R H oder eine Schutzgruppe R₁ bedeutet. Sie können zum Beispiel auf folgende Weise hergestellt werden:

In einer ersten Stufe setzt man 1 Äquivalent Cis-1,3-Pent-4-endiol der Formel A 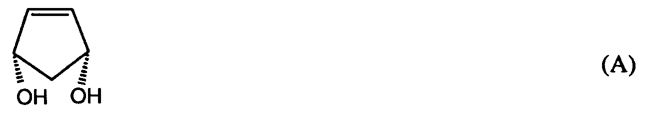
mit einem Äquivalent Di-t-butylsilylditriflat [(t-Butyl)₂Si(CF₃SO₃)₂] zu einer Verbindung der Formel B 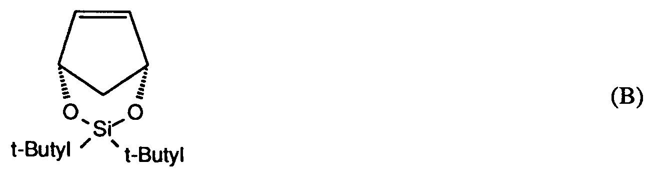
um, die man in einer zweiten Stufe in Gegenwart eines Katalysators , zum Beispiel RhCl[P(C₆H₅)₃]₃, bei erhöhter Temperatur, zum Beispiel 60 bis 150 °C, und unter Druck mit einem Gemisch aus H₂ und CO zu einer Verbindung der Formel C 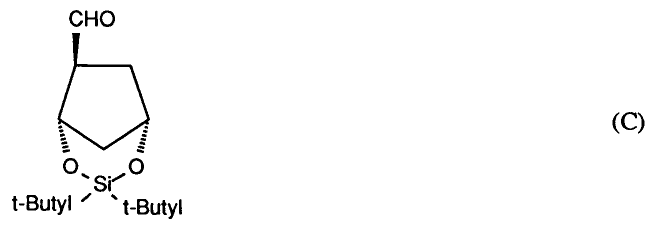
hydroformyliert. Die Verbindung der Formel C wird mit zum Beispiel NaBH₄ oder LiAlH₄ in Gegenwart eines Ethers als Lösungsmittel zu einer Verbindung der Formel D 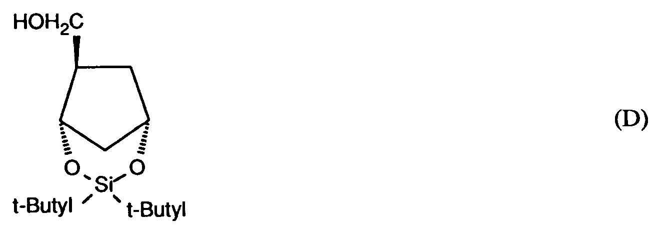
hydriert, und dann die Hydroxylgruppe der Verbindung der Formel D geschützt, zum Beispiel durch Umsetzung mit Tritylchlorid. Aus der geschützten Verbindung der Formel D wird die Brückengruppe (t-Butyl)₂Si= durch Behandeln mit Tetra-n-butylammoniumfluorid-trihydrat abgespalten und man erhält die Verbindungen der Formel E 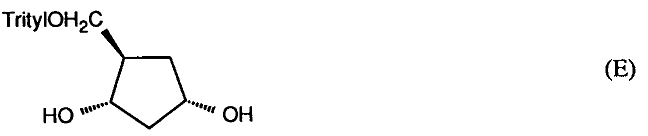
als Racemat. Die Tritylschutzgruppe kann in bekannter Weise abgespalten und durch andere Schutzgruppen ersetzt werden.

Das Racemat kann direkt weiterverwendet oder mittels einer enzymatischen katalysierten Monoacylierung mit Vinylacetat in Gegenwart der Lipase von Pseudomonas Fluorescens oder Chromobakterium viscosum in ein Isomerengemisch mit den Regioisomeren der Formeln F und G übergeführt werden:

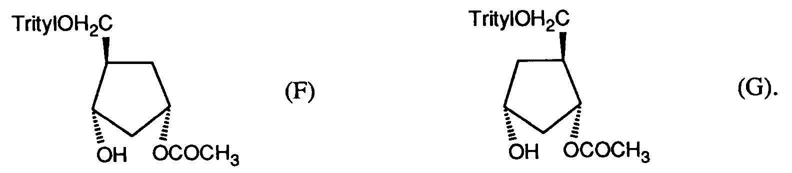

Bei dieser enzymatischen Reaktion werden bevorzugt räumlich anspruchsvolle Schutzgruppe verwendet, zum Beispiel unsubstituiertes oder mit C₁-C₄-Alkyl oder C₁-C₄-Alkoxy substituiertes Diphenylmethyl und besonders Triphenylmethyl (Trityl),

Das erhaltene Isomerengemisch kann in einfacher Weise durch Chromatographie an Kieselgel vollständig getrennt werden, Die Umsetzung der Verbindungen der Formeln F und G mit Basen wie zum Beispiel Ammoniak, Aminen oder Alkalimetallbasen in alkoholischer Lösung führt in praktisch quantitativen optischen Ausbeuten zu den enantiomeren Diolen der Formeln H und J, 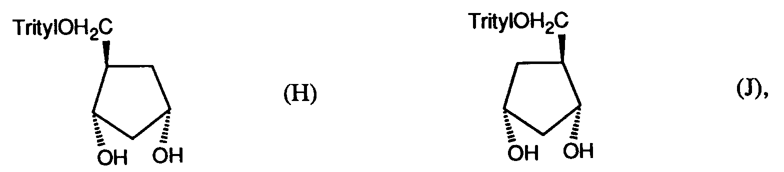
wobei es sich bei den Verbindungen der Formel H um die (+)-Enantiomeren der natürlichen Reihe und entsprechend bei den Verbindungen der Formel J um die (-)-Enantiomeren der unnatürlichen Reihe handelt. Zur Herstellung von Verbindungen der Formeln II, IIa und IIb, worin R H bedeutet, wird die Schutzgruppe in an sich bekannter Weise abgespalten.

Die Reaktion dei Verfahrensstufen a) kann zweckmässig in Gegenwart eines inerten Lösungsmittels durchgeführt werden.

Geeignete inerte Lösungsmittel sind zum Beispiel polare oder unpolare und bevorzugt aprotische Lösungsmittel, die alleine oder in Mischungen aus mindestens zwei Lösungsmitteln verwendet werden können. Beispiele sind: Ether (Dibutylether, Tetrahydrofuran, Dioxan, Ethylenglykoldiethyl- oder -dimethylether, Diethylenglykoldimethylether, Diethylenglykoldiethylether, Triethylenglykoldimethylether), halogenierte Kohlenwasserstoffe (Methylenchlorid, Chloroform, 1,2-Dichlorethan, 1,1,1-Trichlorethan, 1,1,2,2-Tetrachlorethan), Carbonsäureester und Lactone (Essigsäureethylester, Propionsäuremethylester, Benzoesäureethylester, 2-Methoxyethylacetat, γ-Butyrolacton, δ-Valerolacton, Pivalolacton), Carbonsäureamide und Lactame (N,N-Dimethylformamid, N,N-Diethylformamid, N,N-Dimethylacetamid, Tetramethylharnstoff, Hexamethylphosphorsäuretriamid, γ-Butyrolactam, ε-Caprolactam, N-Methylpyrrolidon, N-Acetylpyrrolidon, N-Methylcaprolactam), Sulfoxide (Dimethylsulfoxid), Sulfone (Dimethylsulfon, Diethylsulfon, Trimethylensulfon, Tetramethylensulfon), tertiäre Amine (Triethylamin, N-Methylpiperidin, N-Methylmorpholin), aromatische Kohlenwasserstoffe wie zum Beispiel Benzol oder substituierte Benzole (Chlorbenzol, o-Dichlorbenzol, 1,2,4-Trichlorbenzol, Nitrobenzol, Toluol, Xylol) und Nitrile (Acetonitril, Propionitril, Benzonitril, Phenylacetonitril), sowie aliphatische oder cycloaliphatische Kohlenwasserstoffe (Pentan, Petrolether, Hexan, Cyclohexan und Methylcyclohexan).

Bevorzugte Lösungsmittel sind halogenierte aliphatische Kohlenwasserstoffe, aromatische Kohlenwasserstoffe, Ether und Nitrile, zum Beispiel Methylenchlorid, Chloroform, Benzol, Toluol, Acetonitril, Diethylether, Dibutylether, Tetrahydrofuran und Dioxan.

Die Reaktionstemperatur beträgt in der Verfahrensstufe a) bevorzugt -20 °C bis 200 °C, besonders bevorzugt 0 °C bis 120 °C.

Es hat sich als zweckmässig erwiesen, säurebindende Mittel wie zum Beispiel tertiäre Amine mitzuverwenden, wenn bei der Reaktion Säuren wie zum Beispiel HX gebildet werden. Geeignete tertiäre Amine sind zum Beispiel Trimethyl-, Triethyl-, Tripropyl- oder Tributylamin, Di-i-propylethylamin, N-Methylmorpholin, Pyridin, Collidin und Lutidin. Die säurebindenden Mittel werden vorzugsweise im Überschuss eingesetzt, zum Beispiel bis zu 10 Moläquivalenten, bevorzugt bis zu 5 Moläquivalenten. Bei der Verwendung von X-SO-X als Reaktand kann die gebildete Sulfoxidverbindung ohne Isolierung durch Oxidation mit zum Beispiel Alkalimetallperchloraten, -perbromaten oder -periodaten gegebenenfalls in Gegenwart katalytischer Mengen eines Edelmetallsalzes wie zum Beispiel RuCl₃·H₂O zu den entsprechenden Sulfodioxidverbindungen umgesetzt werden.

Die Isolierung der Verbindungen der Formeln II, IIa und IIb kann, falls gewünscht, nach üblichen Methoden wie zum Beispiel Destillation, Kristallisation und/oder Chromatographie erfolgen.

Die in der Verfahrensstufe a) verwendeten Reaktanden werden im allgemeinen in äquimolaren Mengen eingesetzt. Es hat als vorteilhaft erwiesen, einen Überschuss des zur Einführung der Gruppe Y verwendeten Reaktanden zu verwenden, zum Beispiel bis zu 4, bevorzugt bis zu 2,5 Äquivalente pro Äquivalent der Verbindung der Formeln II, IIa und IIb.

Ein weiterer Gegenstand der Erfindung sind die Racemate der Formel III und die Enantiomeren der Formeln IIIa oder IIIb, 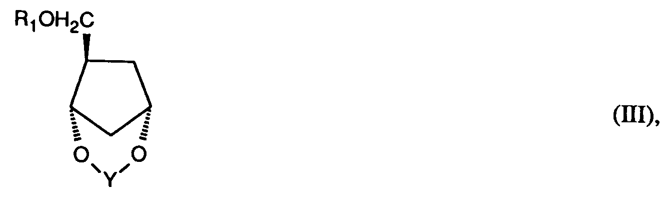 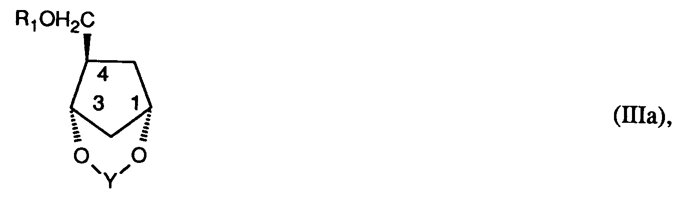 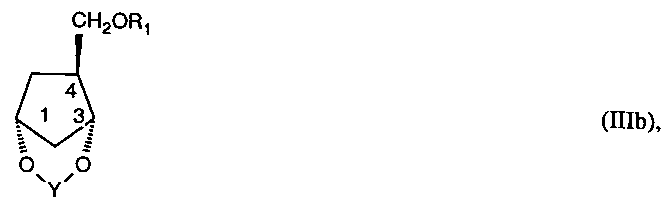
worin Y die Gruppen -C(O)-, -C(S)-, -SO-, -SO₂-, R₂NH-CH=, R₃R₄N-CR₅=, R₆P= und R₆(O)P= darstellt, und R₁ bis R₆ die zuvor angegebenen Bedeutungen haben, einschliesslich der Bevorzugungen. Y steht besonders bevorzugt für die Gruppen -C(O)-, -SO- und -SO₂-.

Die Umsetzung bei der Verfahrensstufe b) wird zweckmässig in Gegenwart eines inerten Lösungsmittels durchgeführt. Geeignete Lösungsmittel sind zuvor genannt worden. Bevorzugt sind polare und aprotische Lösungsmittel, wie zum Beispiel Nitrile, besonders Acetonitril, Propionitril, Butyronitril, Benzonitril und Phenylacetonitril, N,N-dimethylierte oder -diethylierte Carbonsäureamide oder N-methylierte oder -ethylierte Lactame wie zum Beispiel Dimethylacetamid und N-Methylpyrrolidon, Sulfoxide wie zum Beispiel Dimethylsulfoxid oder Sulfone wie zum Beispiel Tetramethylensulfon.

Die Reaktion wird bevorzugt in Gegenwart von Mitteln durchgeführt, die die reaktive NH-Gruppe der Nukleinbase aktivieren und/oder gebildete saure Gruppen -OYOH versalzen. Solche Mittel sind zum Beispiel nicht-nukleophile und bevorzugt lipophile und im Reaktionsmedium lösliche Basen wie zum Beispiel tertiäre Amine mit bevorzugt 3 bis 18 C-Atomen (Trimethylamin, Triethylamin, Tri-n-propylamin, Tri-n-butylamin, N-Methylpiperazin, Azabicycloheptan, Azabycyclohepten, Azabicyclooctan, Azabicycloocten, 1,8-Diaza-bicyclo-[5,4,0]-undec-7-en) oder Disilazane mit bevorzugt C₁-C₄-Alkyl in den Silylgruppen Hexamethyldisilazan), die alleine oder in Mischung eingesetzt werden können. Ferner ist es möglich, die reaktive NH-Gruppe mit Hilfe von zum Beispiel Alkalimetallhydriden, Alkalimetallalkoxiden oder Lithiumalkylverbindungen (zum Beispiel LiH, NaH, KH, LiCH₃ oder LiC₄H₉) zu metallieren. In einer bevorzugten Ausführungsform wird die Umsetzung der Verfahrensstufe b) in Gegenwart von äquimolaren Mengen oder einem geringen Überschuss von 1,8-Diaza-bicyclo-[5,4,0]-undec-7-en durchgeführt.

Die Reaktionstemperatur beträgt bei der Verfahrensstufe b) zum Beispiel von -20 bis 200 °C, bevorzugt 0 bis 150 °C und besonders bevorzugt 10 bis 100 °C.

Die Umsetzung der Verfahrensstufe b) kann zum Beispiel so durchgeführt werden, dass man die Nukleinbase in einem Lösungsmittel suspendiert oder löst, eine nicht-nukleophile Base zugibt und die Suspension rührt, bis eine klare Lösung entsteht. Zu dieser Lösung gibt man dann eine Verbindung der Formeln III, IIIa oder IIIb und lässt ausreagieren. Zur Isolierung der Verbindungen wird das Lösungsmittel verdampft und der Rückstand mit üblichen Methoden, zum Beispiel Chromatographie und Kristallisation, gereinigt. Für die Umsetzung gemäss der Verfahrensstufe c) können auch die Rohprodukte eingesetzt werden. Die Reaktion ist überraschend regiospezifisch und diastereospezifisch und man erhält daher die gewünschten Verbindungen in hohen Ausbeuten und Reinheiten.

Die Abspaltung der Schutzgruppe R₁ und der Gruppe YOM in den Verbindungen der Formeln IV, IVa und IVb kann gleichzeitig oder nacheinander erfolgen, je nach verwendeter Schutzgruppe R₁. Die Gruppe YOM kann hydrolytisch abgespalten werden, zum Beispiel in an sich bekannter Weise durch die Umsetzung mit verdünnten wässrigen Mineralsäuren in alkoholischer Lösung, wobei die Reaktionstemperatur zum Beispiel 20 bis 120 °C betragen kann. Man erhält hierbei mit der Gruppe R₁ geschützte Nukleoside, die je nach verwendeter Nukleosidbase in Form von Salzen der verwendeten Mineralsäuren vorliegen können und die man direkt zur Abspaltung der Schutzgruppe verwenden oder vorher nach üblichen Methoden isolieren kann. Aus den Salzen können die freien Nukleoside durch Behandlung mit Alkalimetallbasen oder mit basischen Ionenaustauschern erhalten werden. Hydrolytisch abspaltbare Schutzgruppen R₁ können gleichzeitig mitabgespalten werden, wobei es zweckmässig sein kann, konzentrierte Mineralsäuren zu verwenden. Geeignete Mineralsäuren sind zum Beispiel wässrige HCl, HBr oder H₂SO₄. Andere Schutzgruppen können nach in der Zuckerchemie allgemein bekannten Methoden entfernt werden

Aus den Verbindungen der Formeln I, Ia und Ib können Oligonukleotide aufgebaut werden, die auf Grund ihrer Wechselwirkung mit Nukleinsäuren wertvolle biologische Aktivitäten aufweisen, und als pharmazeutische Wirkstoffe oder als Diagnostika verwendet werden können. Die Oligonukleotide können gleiche oder verschiedene Monomereinheiten von Verbindungen der Formel I, Ia oder Ib oder Monomereinheiten von anderen Nukleosiden enthalten, wobei die Oligonukleotide 2 bis 200 Monomereinheiten enthalten. Bevorzugt enthalten die Oligonukleotide 2 bis 100, besonders bevorzugt 2 bis 50, und insbesondere bevorzugt 2 bis 20 Monomereinheiten.

Die Herstellung der Oligonukleotide kann in an sich bekannter Weise nach verschiedenen Verfahren in gegebenenfalls automatisierten und zusammen mit Verfahrensvorschriften käuflichen DNA-Synthesizern erfolgen. Im Falle der Brückengruppe -P(O)O^{⊖}- kann zum Beispiel das Phosphortriesterverfahren, das Phosphittriesterverfahren oder das H-Phosphonatverfahren angewendet werden, die dem Fachmann geläufig sind. Beim Phosphittriesterverfahren kann man zum Beispiel so vorgehen, dass man die Nukleoside der Formel I, Ia oder Ib, worin R H bedeutet, mit einem Schutzgruppenreagenz, zum Beispiel 4,4'-Dimethoxytriphenylmethyl-trifluormethylsulfonat (abgekürzt DMT-Triflat) umsetzt, und die erhaltene Verbindung mit Hilfe eines "linkers", zum Beispiel Bernsteinsäureanhydrid, an ein festes Trägermaterial bindet, zum Beispiel an Controlled Pore Glass (CPG), das langkettige Alkylaminogruppen enthält. In einem seperaten Verfahren wird die Hydroxylgruppe der geschützten Verbindung derivatisiert, zum Beispiel zu einem Phosphoramidit unter Verwendung von [R'O(i-Propyl₂N)]PCl, wobei R' zum Beispiel Allyl oder β-Cyanoethyl darstellt. Nach dem Abspalten der Schutzgruppe des an den Träger gebundenen Materials koppelt man unter Abspaltung von -N(i-C₃H₇) mit dem geschützten Nukleosid, blockiert eventuell vorhandene freie Hydroxylgruppen (capping) und oxidiert dann das gebildete Phosphit zum Phosphat. Nach dem Entschützen des Dimeren wiederholt man den Reaktionszyklus mit dem geschützten Nukleosid, bis man ein Oligomer mit der gewünschten Anzahl an Monomereinheiten synthetisiert hat, und löst das Produkt vom Trägermaterial ab.

Die Verbindungen der Formel I, Ia und Ib, worin R H bedeutet, weisen antivirale und antiproliferative Eigenschaften auf und können demgemäss als Arzneimittel Verwendung finden. Die Oligonukleotide weisen eine gute Stabilität gegenüber einem Abbau durch Nukleasen (zum Beispiel Enzyme) auf. Ferner wird eine sehr gute Paarung mit Nukleinsäuren, besonders doppelsträngigen Nukleinsäuren unter Ausbildung von stabilen Trippelhelices beobachtet. Die Oligonukleotide eignen sich daher besonders für die Antisense-Technologie zur Inaktivierung von Nukleosidsequenzen in Nukleinsäuren (siehe EP-A-0 266 099, WO 87/07300 und WO 89/08146). Sie können zur Behandlung von Infektionen und Krankheiten zum Beispiel durch die Blockierung von bioaktiven Proteinen auf der Stufe der Nukleinsäuren (zum Beispiel Onkogene) eingesetzt werden. Die Oligonukleotide eignen sich auch als Diagnostika und können als Gensonden zum Nachweis von viralen Infektionen oder genetisch bedingten Krankheiten durch selektive Interaktion auf der Stufe von einzel- oder doppelsträngigen Nukleinsäuren verwendet werden ("gene probes"). Im besonderen - bedingt durch die sehr gute Stabilität gegenüber Nukleasen - ist eine diagnostische Anwendung nicht nur *in vitro* sondern auch *in vivo* (zum Beispiel Gewebeproben, Blutplasma und Blutserum) möglich. Solche Verwendungsmöglichkeiten sind zum Beispiel in der WO 91/06556 beschrieben.

Die nachfolgenden Beispiele erläutern die Erfindung.

### A) Herstellungsbeispiele für Ausgangsverbindungen

### Beispiel A1: Herstellung von

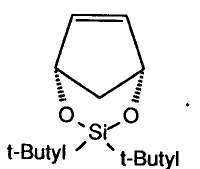

0,91 g (9,1 mMol) cis-4-Cyclopenten-1,3-diol und 20 ml wasserfreies Methylenchlorid werden unter Argonatmosphäre in einem Reaktionskolben vorgelegt und auf 0 °C gekühlt. Unter Rühren werden 3,17 ml (27,3 mMol) Lutidin zugegeben und danach innerhalb von 10 Minuten 4,42 g (10 mMol) Di-t-butylsilylditriflat. Das Reaktionsgemisch wird 10 Minuten bei 0 °C gerührt, dann das Eisbad entfernt und 30 Minuten lang weitergerührt. Danach entfernt man das Lösungsmittel unter Vakuum und versetzt den öligen Rückstand unter kräftigem Rühren mit 20 ml Hexan. Das gebildete kristalline Lutidiniumtriflat wird abfiltriert das Lösungsmittel aus dem Filtrat verdampft. Man chromatographiert den Rückstand an Silikagel mit Hexan/Diethylether (20:1) und erhält nach Abdampfen des Lösungsmittels und Trocknen des Rückstands (Raumtemperatur, 30 Minuten, 1,3 Pa) 1,4 g (65 %) eines farblosen Öls. ¹H-NMR (250 MHz, CDCl₃): 0,96 und 1,04 (2 s, 2 (CH₃CSi); 1,81 [dt, J=12,0 und 3,0, H-C(2)]; 2,49 [d, J=12,0, HC(2)].

Eine Wiederholung des Verfahrens mit der fünffachen Menge ergibt eine Ausbeute von 80 % der Theorie.

### Beispiel A2: Herstellung von

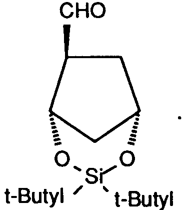

1 g (4,16 mMol) der Verbindung gemäss Beispiel A1, 15,4 mg (0,4 Mol-%) RhCl[P(C₆H₅)₃]₃ und 30 ml Tetrahydrofuran werden in einem 50 ml goldbeschichteten Autoklaven vorgelegt und dann unter einer H₂/CO-Atmosphäre bei 8 MPa und 80 °C 5 Stunden lang erhitzt. Nach dem Abkühlen wird das Lösungsmittel im Vakuum abgedampft und der Rückstand an Silikagel chromatographiert (Hexan/Essigsäureethylester 9:1). Danach entfernt man die Lösungsmittel im Vakuum und trocknet den Rückstand eine Stunde bei Raumtemperatur und 8 Pa. Man erhält 1,07 g (95 %) der Titelverbindung als farbloses Öl. ¹H-NMR (250 MHz, CDCl₃): 1,05 und 1,06 (2s, 2 (CH₃CSi); 1,24 [ddd, J=14,0, 3,0 und 3,0, H-C(2)]; 2,44 [d, J=14,0, HC(2)]; 9,79 (s, HCO).

### Beispiel A3: Herstellung von

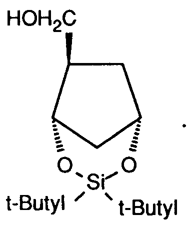

491 mg (1,82 mMol) der Verbindung gemäss Beispiel A2 werden in 10 ml Tetrahydrofuran/H₂O (9:1) gelöst und auf 0 °C gekühlt. Unter Rühren werden dann 34,5 mg (0,91 mMol) NaBH₄ auf einmal zugegeben und noch 10 Minuten weitergerührt. Danach wird das Lösungsmittel verdampft, der Rückstand zweimal mit je 30 ml Essigsäureethylester extrahiert, und die organische Phase mit je 20 ml 2,5-prozentiger wässriger NaHCO₃ und konzentrierter wässriger Kochsalzlösung gewaschen. Die organische Phase wird dann mit Na₂SO₄ getrocknet, filtriert und das Filtrat zur Trockne eingedampft. Nach einem einstündigen Trocknen des Rückstandes im Hochvakuum bei Raumtemperatur erhält man 485 mg (98 %) der Titelverbindung als farbloses Öl, das noch eine geringe Menge Essigsäureethylester enthält. ¹H-NMR (300 MHz, CDCl₃): 1,04 und 1,05 (2s, 2 CH₃CSi); 1,38 (ddd, J=14,5, 5,0 und 5,0) und 1,47 (ddd, J=3,5, 3,5 und 13,5) und 2,30 (ddd, J=3,0, 8,5 und 14,5) und 2,42 (d, J=13,5), (H₂-C(2) und H₂C(5)]; 1,59 [s, HOCH₂); 3,36 [dd, J=8,5 und 10,5, HOH₂C(6)].

### Beispiel A4: Herstellung von

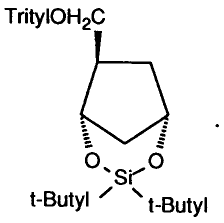

397 mg (1,46 mMol) der Verbindung gemäss Beispiel A3, 17,8 mg (0,15 mMol) 2-Dimethylaminopyridin, und 405 µl (2,91 mMol) Triethylamin werden unter Argonatmosphäre in 5 ml CH₂Cl₂ gelöst. Dann werden unter Rühren der Lösung bei Raumtemperatur 528 mg (1,89 mMol) Tritylchlorid auf einmal zugegeben und 18 Stunden weitergerührt. Danach wird das Lösungsmittel im Vakuum abgedampft und der Rückstand mit zweimal 50 ml Hexan/Essigsäureethylester extrahiert. Die Lösung wird darauf mit je 30 ml wässriger 6-prozentiger NaHCO₃ und konzentrierter Kochsalzlösung gewaschen, mit Na₂SO₄ getrocknet, abfiltriert, das Lösungsmittel im Vakuum abgedampft und der Rückstand im Hochvakuum bei Raumtemperatur über Nacht getrocknet. Man erhält 657 mg (88 %) der Titelverbindung. ¹H-NMR (250 MHz, CDCl₃): 1,04 und 1,05 (2s, 2 (CH₃CSi); 1,32-1,50 (m) und 2,22 (ddd, J=14,5, 3,0 und 9,0) und 2,35 (d, J=13,5), (H₂-C(2) und H₂C(5)]; 2,66 [m, HC(4)], 4,43-4,58 [m, H-C(1,3)]; 7,17-7,47 [m, 15H-C(aromatisch)].

### Beispiel A5: Herstellung von

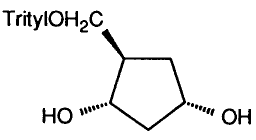

als Racemat.

Eine Lösung von 752 mg (2,39 mMol) Tetra-n-butylammoniumfluorid-trihydrat in 10 ml Tetrahydrofuran wird zu 558 mg (1,08 mMol) der Verbindung gemäss Beispiel A4 gegeben und 5 Stunden bei Raumtemperatur gerührt. Man dampft danach das Lösungsmittel im Vakuum ab, löst den Rückstand in Essigsäureethylester/Hexan (2:1) und chromatographiert die Lösung an Silikagel. Von den gesammelten Fraktionen wird das Lösungsmittel im Vakuum abgedampft und dann der Rückstand im Hochvakuum bei Raumtemperatur während 18 Stunden getrocknet. Man erhält 409 mg (quantitative Ausbeute) der Titelverbindung als farblose und harzige Substanz, die beim Stehenlassen kristallisiert. Die Umkristallisation aus Essigsäureethylester/Hexan (1:2) ergibt eine kristalline Substanz mit einem Schmelzpunkt von 116-117 °C. ¹H-NMR (250 MHz, CDCl₃): 1,48 (ddd, J=5,5, 8,8 und 14,0) und 1,78 (m, 2 Hauptsignale) und 1,88-2,08 (m) [H₂-C(2) und H₂-C(5)]; 2,51 [H-C(4)]; 2,03 (d,J=5,0) und 2,58 [d, J=5,0, HO-C(1,3)]; 2,93 [dd, J=8,5 und 8,5, H-C(6)]; 3,21 [dd, J=5,5 und 9,0, H-C(6)]; 4,09 und 4,32 [2m, H-C(1,3)]; 7,18-7,46 [m, H-C(aromatisch)].

### Beispiel A6: Herstellung von

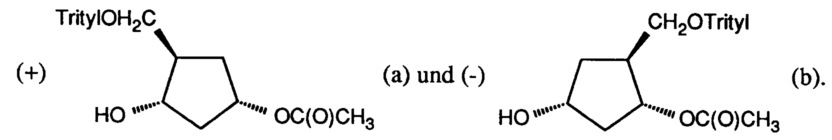

6,22 g (16,6 mMol) der Verbindung gemäss Beispiel A5 werden in 63 ml Vinylacetat gelöst und mit 1,24 g Pseudomonas fluorescens Lipase (PFL) versetzt. Die heterogene Reaktionsmischung wird darauf 50 Stunden bei Raumtemperatur gerührt. Danach wird die Mischung im Vakuum eingedampft, in Hexan/Essigsäureethylester (2:1) aufgenommen und dann an Silikagel chromatographiert. Man erhält 250 mg der Diacetatverbindung, 3,38 g reine Verbindung (a) und 3,45 g reine Verbindung (b) sowie 970 mg nicht getrennter Monoacetate, die nochmals chromatographiert werden. Die vereinigten reinen Verbindungen (a) und (b) werden dreimal in CH₃CN gelöst und im Vakuum zur Trockene eingedampft. Man erhält 3,00 g (43,4 %) der Verbindung (a) und 3,15 g (45,5 %) der Verbindung (b) als leicht gelbe Öle, die noch mit etwas CH₃CN verunreinigt sind.
¹H-NMR (250 MHz, CDCl₃) der Verbindung (a), (1S,3S,4R)-Trans-4-trityloxy-cis-3-hydroxy-1-acetoxy-cyclopentan: 1,54 [m, H-C(5)]; 1,74 [m, H₂C(2)]; 1,89 [m, H-C(5)]; 2,05 (s,H₃CCOO); 2,40 [m, H-C(2) und H-C(4)]; 2,52 [d, J=4,0, HO-C(3)]; 2,98 [t, J=8,5, H-C(6)]; 3,34 [dd, J=9,0 und 5,0, H-C(6)]; 3,95 [dq, J=3,0 und 7,0, H-C(3)]; 5,07 [m, H(1)]; 7,21-7,45 [m, H-C(aromarisch)]. Die Verbindung (a) wird mit einer optischen Reinheit von ee gleich 98,2 % erhalten (bestimmt mit HPLC, Chiracel® OD, Hexan/i-Propanol 9:1, Fliessrate 1 ml/Minute).
¹H-NMR (250 MHz, CDCl₃) der Verbindung (a), (1R,3R,4S)-Trans-4-trityloxy-cis-1-hydroxy-3-acetoxy-cyclopentan: 1,61-178 [m, H-C(2,5) und HO-C(1)]; 1,95 [m, H-C(5)]; 2,03 [s, H₃-CCOO]; 2,30 [ddd, J=14,5 und 7,5 und 5,5, H-C(2)]; 2,60 [m, H-C(4)]; 3,12 [ABM-System, H₂-C(6)]; 4,34 [m, H-C(1)]; 5,05 [m, H-C(3)]; 7,18-7,45 [m, H-C(aromatisch)]. Die Verbindung (b) wird mit einer optischen Reinheit von ee 98,6 % erhalten.

### Beispiel A7: Herstellung von

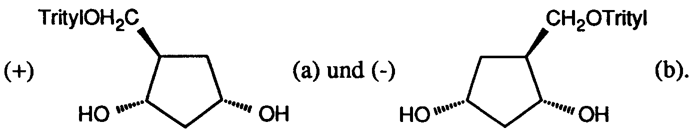

100 g (240 mmol) der Monoacetate gemäss Beispiel A6 werden unter Argon in 600 ml Methanol gelöst und 188,5 g (4,8 mol) Ethylendiamin zugegeben, wobei die Temperatur auf 50 °C ansteigt. Das Reaktionsgemisch wird 15 h bei dieser Temperatur gerührt. Danach wird das Lösungsmittel im Hochvakuum abgedampft und der Rückstand dreimal in Acetonitril aufgenommen und wieder eingedampft. Das erhaltene Öl wird in 500 ml Ethylacetat gelöst, 300 ml Wasser zugegeben und unter Rühren wässrige Zitronensäurelösung (10 %) zugefügt. Nach Zugabe von weiteren 500 ml Ethylacetat wird die organische Phase abgetrennt und die wässrige Phase zweimal mit je 150 ml Ethylacetat extrahiert. Die vereinigten organischen Phasen werden über NaHCO₃ getrocknet und dann eingedampft. Der Rückstand wird zweimal in Acetonitril aufgenommen und eingedampft und dann aus Cyclohexan (1 Woche, 8-10 °C) umkristallisiert. Nach dem Trocknen erhält man je 85,2 g (95 %) der Titelverbindungen als farblose Kristalle. Die ¹H-NMR-Spektren sind mit dem Racemat gemäss Beispiel B1 identisch.
Verbindung (a): Schmelzpunkt 105-106 °C; [α]²⁵= 24,1 (589, c = 1,0, CH₃OH), +73,7 (365 nm). Verbindung (b): Schmelzpunkt 105-106 °C; [α]²⁵= -25,8 (589, c = 1,0, CH₃OH), -75,2 (365 nm).

### B) Herstellung von Nukleosiden

### Beispiel B1: Herstellung von

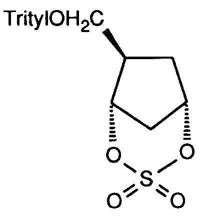

als Racemat.
a) 1,049 g (2,80 mMol) der Verbindung gemäss Beispiel A5 werden unter Argonatmosphäre in 20 ml CH₂Cl₂ und 1,56 ml (11,4 mMol) Triethylamin gelöst. Die Lösung wird unter Rühren auf 0 °C gekühlt und 302 µl (4,2 mMol) SOCl₂ während 5 Minuten zugetropft. Die Reaktionsmischung ändert ihre Farbe von farblos über gelb in braun und nach 10 Minuten fällt ein brauner Niederschlag aus. Darauf wird das Reaktionsgemisch zweimal mit 20 ml eiskaltem Wasser und dann mit 30 ml eiskalter Kochsalzlösung (18 Volumen-%) extrahiert. Die organische Phase wird dann über MgSO₄ getrocknet, filtriert, und bei 15 °C im Vakuum zur Trockne eingedampft. Der Rückstand wird zweimal in je 6 ml CH₃CN gelöst, wieder zur Trockne eingedampft und im Hochvakuum 10 Minuten getrocknet. Man erhält 1,335 g des cyclischen Sulfits (113 %, Gehalt an CH₃CN und Triethylamin als Verunreinigungen), die so im nachfolgenden Verfahren b) verwendet werden.
b) 8,50 g der nach a) hergestellten Verbindung werden in 160 ml CH₃CN/CCl₄ (1:1) gelöst und auf 0 °C gekühlt. Dann fügt man nacheinander 120 ml Wasser, 47 mg (200 µmol) RuCl₃xH₂O und 5,71 g (26,7 mMol) NaIO₄ zu. Die Mischung wird während einer Stunde kräftig gerührt, danach 300 ml Diethylether zugefügt und die wässrige Phase abgetrennt. Die organische Phase wird dreimal mit 100 ml eiskalter und konzentrierter wässriger Kochsalzlösung gewaschen, über MgSO₄ getrocknet, abfiltriert, im Vakuum bei 15 °C eingedampft und dann im Hochvakuum getrocknet. Man erhält 5,80 g (99,5 %, bezogen auf die beiden Verfahrensstufen) der Titelverbindung als farblosen und amorphen Feststoff. ¹H-NMR (250 MHz, CDCl₃): 1,61 (dt, J=13,5 und 4,0) und 1,77 (dt, J=12,5 und 2,5) und 2,69 (d, J=12,5) und 2,80-2,92 (m, teilweise überlappende Signale) H₂-C(2) und H₂-C(5)]; 3,39 [m, H-C(4)]; 2,84 (dd, J=8,5 und 7,5,) und 3,29 [dd, J=8,5 und 4,5, H₂-C(6)]; 5,09 (breites s) und 5,22 [breites s, H-C(1,3)]; 7,21-7,45 [m, H-C(aromatisch)].

Auf die gleiche Weise erhält man unter Verwendung von enantiomeren Cyclopentandiolen die enantiomeren cyclischen Sulfate, zum Beispiel das (+)-Enantiomere mit einem [α]²⁵ gleich +9,2 bei 589 nm (c = 1,05, CHCl₃).

### Beispiel B2: Herstellung von

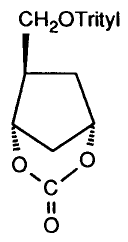

als Racemat.

200 mg (534 µmol) der Verbindung gemäss Beispiel A5 werben in Toluol aufgeschlämmt, wenig Molekularsieb (3Å) zugegeben und etwa 5 min unter Argon gerührt. 5 min nach der Zugabe von 95,3 mg (588 µmol) Carbonyldiimidazol wird die Reaktionsmischung klar. Nach 36 h Rühren bei 90°C und 45 Tagen bei bei Raumtemperatur wird am Rotationsverdampfer eingeengt und das Reaktionsgemisch an Kieselgel gereinigt (Essigsäureethylester/Hexan 1:2). Die reinen Produktfraktionen werden vereinigt und eingeengt, dann in CH₃CN aufgenommen und erneut eingeent. Man erhält 53 mg (25%) reines Produkt. ¹H-NMR (250 MHz, CDCl₃): u.a. 4,84 (m) und 4,78 (m)(H-C(1,3)); 3,22 (quartettartiges m, H-C(4)); 2,84 (m, CH₂OTr).

### Beispiel B3: Herstellung von (±)-Cis-4-hydroxymethyl-1-(9-adenyl)-trans-3-hydroxy-cyclopentan:

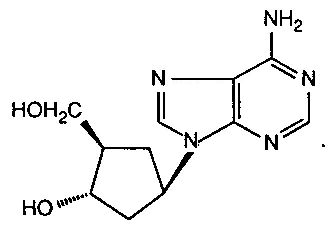

a) 297 mg (2,20 mMol) Adenin werden unter Argonatmosphäre in 10 ml CH₃CN suspendiert und dann 337 mg (2,21 mMol) 1,8-Diaza-bicyclo-[5,4,0]-undec-7-en (DBU) zugegeben. Man rührt die Mischung 15 Minuten bei Raumtemperatur und versetzt anschliessend mit 0,50 g (1,15 mMol) des Racemats gemäss Beispiel B2. Danach wird die Reaktionsmischung noch 15 Stunden bei Raumtemperatur gerührt. Nach dem Abdampfen des Lösungsmittels im Vakuum wird der Rückstand in CH₂Cl₂/Methanol/Triethylamin (30:3:1) aufgenommen und an Silikagel chromatographiert. Die vereinten Fraktionen werden zur Trockne eingedampft und der Rückstand zweimal in je 20 ml CH₃CN gelöst und wieder zur Trockne eingedampft. Der Rückstand wird dann in 30 ml Wasser gelöst und mit Triethylamin versetzt. Der Niederschlag wird abfiltriert und getrocknet. Man erhält 590 mg (76%) des Tetraethylammoniumsalzes von (±)-Cis-4-Trityloxymethyl-1-(9-adenyl)trans-3-sulfato-cyclopentan als farbloses Pulver. ¹H-NMR (250 MHz, CD₃OD): 1,31 [t, J=7,0, (CH₃CH₂)₃NH⁺]; 3,20 [q, J=7,0, (CH₃CH₂)₃NH⁺]; 1,64-2,66 [m, H₂-C(2,5) und H-C(4)]; 3,15-3,40 [m, H₂-C(6)]; 5,02-5,17 [m, H-C(1,3)]; 7,18-7,51 [m, H-C(Trityl)]; 8,15 und 8,16 [2s, H-C(Adenin)].
b) 200 mg der gemäss a) hergestellten Verbindung werden in je 10 ml 2N HCl und Ethanol gelöst und 24 Stunden bei 80-85 °C erhitzt. Danach wird das Lösungsmittel im Vakuum abgedampft und der Rückstand zweimal in je 20 ml CH₃CN aufgenommen und eingedampft. Das erhaltene Rohprodukt wird in Methanol/Essigsäureethylester (1:1) aufgenommen und an Silikagel chromatographiert. Nach dem Abdampfen des Lösungsmittels wird der Rückstand mit dem gleichen Lösungsmittel umkristallisiert. Man erhält 80,1 mg (94 %) (±)-Cis-4-Hydroxymethyl-1-(9-adenyl)-trans-3-hydroxy-cyclopentan-Hydrochlorid als farblose Kristalle. ¹³C-NMR (63 MHz, CD₃OD): 35,1 (C(2')), 41,7 (C(6')), 50,7 (C(4')), 55,9 (C(1')), 64,2 (C(5')), 73,7 (C(3')), 120,3, 150,3 and 151,7 (C(4,5,6)), 144,3 and 144,7 (HC(2,8)).
c) 400 mg (1,40 mmol) der gemäss b) erhaltenen Verbindung wird in Methanol gelöst und über eine Kolonne mit 10 g Amberlit IRA-93 (OH-Form) filtriert. Die gesammelten Fraktionen werden im Vakuum eingedampft und zweimal mit CH₃CN aufgenommen und eingedampft. Der Rückstand wird in Methanol gelöst, zum Rückfluss erhitzt und Essigsäuremethylester zugegeben, bis die Mischung leicht trüb wird. Dann lässt man über Nacht bei 4 °C kristallisieren, filtriert die Kristalle ab und trocknet im Hochvakuum. Man erhält 320 mg (92%) der Titelverbindung als farblose Kristalle mit einen Schmelzpunkt von 183 bis 185 °C. ¹H-NMR (250 MHz, CD₃OD): 1,94 (dt, J = 12,5, 10,0) and 2,14-2,66 (m)(H₂-C(2',6') and H-C(4')); 3,72 (ABM-system, H₂-C(5')); 4,32 (m, H-C(3')); 5,24 (quint.-like m, H-C(1')); 8,41 (s) and 8,48 (s)(H-C(2,8)).

### Beispiel B4: Herstellung von (1R,3S,4R)-Cis-4-hydroxymethyl-trans-3-hydroxy-1-(9'-adenyl)-cyclopentan.

Die Verbindung (a) von Beispiel A7 wird gemäss Beispiel B1 in das cyclische Sulfat übergeführt und 11,50 g (26,3 mmol) gemäss Beispiel B3 mit 3,76 g (27,8 mmol) Adenin umgesetzt. Man erhält 14,25 g (80,4 %) der kristallinen Titelverbindung. ¹H-NMR (250 MHz, CD₃OD): 1,94 (dt, J = 12,5, 10,0) and 2,14-2,66 (m)(H₂-C(2',6') and H-C(4')); 3,72 (ABM-system, H₂-C(5')); 4,32 (m, H-C(3')); 5,24 (quintettartiges m, H-C(1')); 8.41 (s) und 8,48 (s)(H-C(2,8)).

### Beispiel B5: Herstellung von (1S,3R,4S)-Cis-4-hydroxymethyl-trans-3-hydroxy-1-(9'-adenyl)-cyclopentan.

Es wird gemäss Beispiel B4 verfahren, aber mit Verbindung (b) von Beispiel A7. Man erhält 76,9 % der Titelverbindung als farblose Kristalle, Schmelzpunkt 185-186 °C, [α]²⁵= -10,2 (589, c = 0,5, H₂O).

### Beispiel B6: Herstellung von (1S,3R,4S)-Cis-4-hydroxymethyl-trans-3-hydroxy-1-(2'-chlor-6'-aminopurin-9'-yl)-cyclopentan.

Es wird mit der Verbindung b von Beispiel A7 unter Verwendung von 2-Chlor-6-aminopurin gemäss Beispiel B4 verfahren, wobei je 2,0 Moläquivalente 6-Amino-2-chlorpurin und DBU eingesetzt werden und die Reaktionsdauer 15 h bei 50 °C beträgt. Die Hydrolyse wird bei 80 °C in einer 1:1-Mischung aus Ethanol und 2N HCl während 20 h durchgeführt. Die Ausbeute beträgt bei der ersten Verfahrensstufe 30,8 % und bei der zweiten Verfahrensstufe 63 %. ¹H-NMR (300 MHz, CD₃OD): u.a. 5,07 (quintettartiges m, H-C(1)). ¹³C-NMR (CD₃OD): u.a. 55,1 (C(1)); 64,4 (C(5)); 73,8 (C(3)); 141,5 (C(8')).

### Beispiel B7: Herstellung von (1R,3S,4R)-Cis-4-hydroxymethyl-trans-3-hydroxy-1-(9'-guanyl)-cyclopentan-Hydrochlorid.

Es wird unter Verwendung von 2-Amino-6-chlorpurin gemäss Beispiel B4 verfahren, wobei in der ersten Verfahrensstufe die Reaktionsdauer bei Raumtemperatur 3 h beträgt und nach Chromatographie an Kieselgel (CH₂Cl₂/Methanol/Triethylamin 50:30:1) das amorphe Sulfat in 75,3 % Ausbeute erhalten wird. Die zweite Verfahrensstufe wird 20 h bei 70 °C durchgeführt. Man erhält auf diese Weise nach chromatographischer Reinigung (Essigsäureethylester/Methanol/N-Methylmorpholin 25:25:1) amorphes carbacyclisches 2'-Deoxyguanosin in 78.4% Ausbeute, welches in Methanol/konz. HCl (5:1) aufgenommen und am Rotationsverdampfer zur Trockene eingedampft wird. Kristallisation aus Methanol/Essigsäuremethylester liefert die Titelverbindung in einer Ausbeute von 68% mit Schmelzpunkt von 247-248 °C (Zersetzung). ¹H-NMR (300 MHz, DMSO-d₆): u.a. 4,89 (quintettartiges m, H-C(1)). ¹³C-NMR (DMSO-d₆): u.a. 54,1 (C(1)); 62,5 (C(5)); 71,2 (C(3)); 135,6 (C(8')).

### Beispiel B8: Herstellung von (1R,3S,4R)-Cis-4-hydroxymethyl-trans-3-hydroxy- 1-(2',6'-diaminopurin-9-yl)-cyclopentan-Hydrochlorid.

10,0 g (14,1 mMol) des amorphen 2-Amino-6-chlorpurincyclopentan-Sulfats von Beispiel B7 werden in 500 ml Methanol gelöst, die Lösung in einen Autoklaven gepresst und bei 36 °C 11 bar Ammoniak aufgepresst. Danach lässt man 15 h bei 130 °C reagieren. Das Lösungsmittel und der Ammoniak werden dann entfernt, der Rückstand dreimal in Acetonitril aufgenommen und bis zur Trockne eingedampft. Man erhält 9,91 g (101,9 %) 2,6-Diaminopurincyclopentan-Sulfat. Das Rohprodukt wird ungereinigt in der folgenden Hydrolysestufe eingesetzt. Die Hydrolyse erfolgt gemäss Beispiel B4 während 15 h bei Raumtemperatur. Das Reaktionsprodukt wird chromatographisch an Kieselgel (Essigsäureethylester/Methanol 7:3) gereinigt. Man erhält 82,6 % der Titelverbindung, die etwa 5-10 % Verunreinigungen erhält. ¹H-NMR (250 MHz, DMSO-d₆): u.a. 5,06 (quintettartiges m, H-C(1)). ¹³C-NMR (DMSO-d₆): u.a. 52,5 (C(1)); 62,6 (C(5)); 71,4 (C(3)); 139,0 (C(8')).

### Beispiel B9: Herstellung von (1R,3S,4R)-Cis-4-hydroxymethyl-trans-3-hydroxy-1-(1'-cytidyl)-cyclopentan.

Es wird unter Verwendung von Isobutyrocytosin gemäss Beispiel B4 verfahren bei 1 h Reaktionsdauer und 80 °C. Nach Chromatographie an Kieselgel (CH₂Cl₂/MeOH/Et₃N 90:3:1) wird das amorphe Triethylammoniumsalz des tritylgeschützten Isobutyrocyridylsulfats in 61.3% Ausbeute erhalten. ¹H-NMR (250 MHz, CD₃OD): u.a. 5,04 (quintettartiges m, H-C(1)). Die Hydrolyse wird 20 h bei Raumtemperatur durchgeführt. Man erhält nach chromatographischer Reinigung (Essigsäureethylester/Methanol/NH₃aq. 7:3:1) 86.5% der Titelverbindung. Schmelzpunkt 204-205 °C. ¹H-NMR (300 MHz, CD₃OD): u.a. 5,08 (quintettartiges m, H-C(1)). ¹³C-NMR (CD₃OD): u.a. 57,5 (C(1)); 64,2 (C(5)); 73,6 (C(3)); 146,3 (C(6')).

### Beispiel B10: Herstellung von (1R,3S,4R)-Cis-4-hydroxymethyl-trans-3-hydroxy-1-(1'-thymidyl) -cyclopentan.

Es wird unter Verwendung von Methylthymin gemäss Beispiel B4 verfahren. Nach Chromatographie an Kieselgel (CH₂Cl₂/Methanol/Triethylamin 50:3:1) wird amorphes tritylgeschütztes Thymidylcyclopentan-Sulfat in 70,4% Ausbeute erhalten. ¹H-NMR (250 MHz, DMSO-d₆): u.a. 4,49 (m, H-C(3)) und 4,89 (quintettartiges m, H-C(1)). Nach der Hydrolyse (15 h, 45 °C) und chromatographischen Reinigung (Essigsäureethylester/Methanol 7:1) erhält man 79.2% der Titelverbindung als farblose Kristalle, Schmelzpunkt 175-176 °C. ¹H-NMR (300 MHz, CD₃OD): u.a. 5,06 (quintettartiges m, H-C(1)). ¹³C-NMR (CD₃OD): u.a. 55,9 (C(1)); 64,3 (C(5)); 73,7 (C(3)); 139,9 (C(6')).

## Patentansprüche

1. Verfahren zur Herstellung von racemischen Verbindungen der Formel I 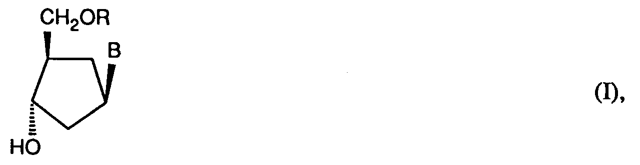 oder der Enantiomeren der Formeln (Ia) und (Ib) 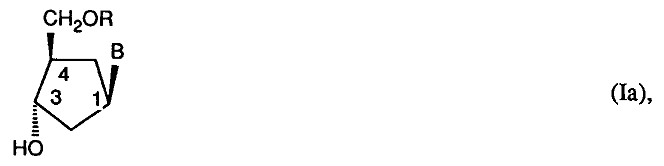 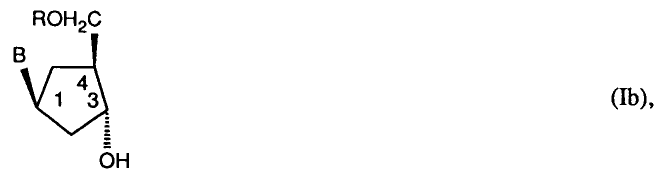 worin B für den Rest einer Nukleinbase aus der Reihe Purin oder einem Purinanalogen oder pyrimidinanalogen steht und R H oder eine Schutzgruppe R₁ bedeutet, dadurch gekennzeichnet, dass man
a) eine Verbindung der Formel II in Form des Racemats oder deren Enantiomeren der Formeln IIa oder IIb 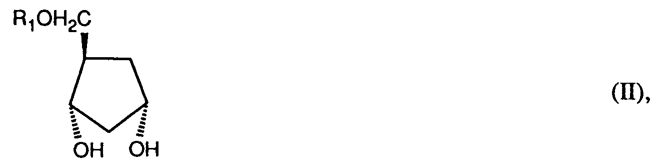 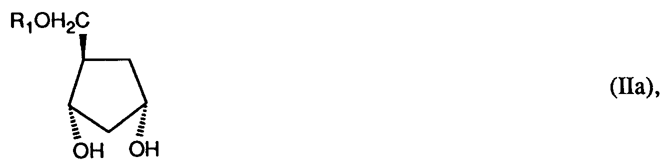 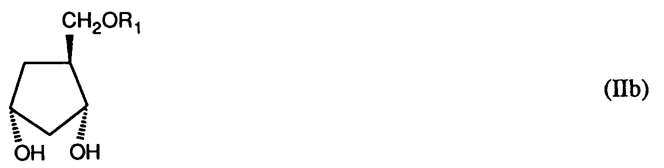 worin R₁ für eine Schutzgruppe steht, mit einer Verbindung der Formeln X-CO-X, X-CS-X, X-SO-X, X-SO₂-X, R₂-NCO, R₃R₄N-C(O)-R₅, X₂P-R₆ oder X₂P(O)-R₆, worin X eine Abgangsgruppe bedeutet, R₂ für lineares oder verzweigtes C₁-C₁₈-Alkyl, C₅-C₈-Cycloalkyl, Phenyl, Benzyl, oder mit C₁-C₆-Alkyl oder C₁-C₆-Alkoxy substituiertes Phenyl oder Benzyl steht, R₃ und R₄ unabhängig voneinander H, lineares oder verzweigtes C₁-C₁₈-Alkyl, C₅-C₈-Cycloalkyl, Phenyl, Benzyl, oder mit C₁-C₆-Alkyl oder C₁-C₆-Alkoxy substituiertes Phenyl oder Benzyl, oder R₃ und R₄ zusammen Tetramethylen, Pentamethylen oder 3-Oxa- 1,5-pentylen bedeuten, R₅ für H, lineares oder verzweigtes C₁-C₁₂-Alkyl, C₅-C₈-Cycloalkyl, Phenyl, Benzyl, oder mit C₁-C₆-Alkyl oder C₁-C₆-Alkoxy substituiertes Phenyl oder Benzyl steht, und R₆ lineares oder verzweigtes C₁-C₁₂-Alkyl oder -Alkoxy, C₅-C₈-Cycloalkyl oder -Cyclalkoxy, Phenyl oder Phenyloxy, Benzyl oder Benzyloxy, oder mit C₁-C₆-Alkyl oder C₁-C₆-Alkoxy substituiertes Phenyl oder Phenyloxy oder Benzyl oder Benzyloxy darstellt, zu Racematen der Formel III oder Enantiomeren der Formeln IIIa oder IIIb umsetzt, 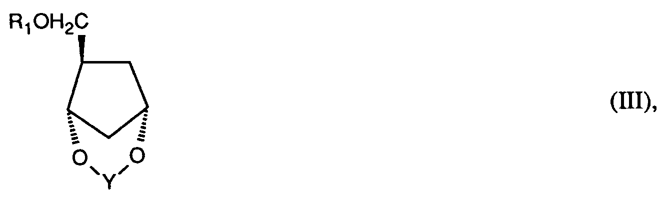 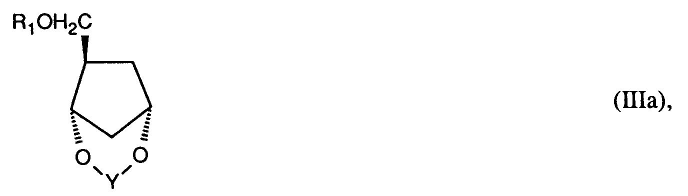 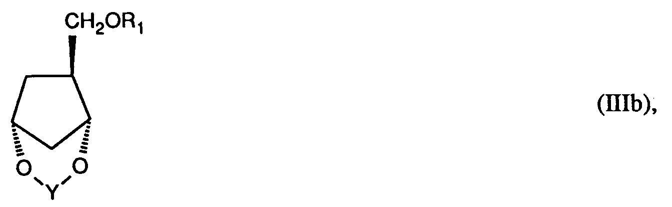 worin Y die Gruppen -C(O)-, -C(S)-, -SO-, -SO₂-, R₂NH-CH=, R₃R₄N-CR₅=, R₆P= und R₆(O)P= darstellt, worin R₁ und R₂ bis R₆ die zuvor angegebenen Bedeutungen haben,
b) die Verbindungen der Formeln III, IIIa oder IIIb mit einer Nukleinbase B, gegebenenfalls zusammen mit einer nicht-nukleophilen Base, oder Alkalimetallsalzen einer Nukleinbase B zu Racematen der Formeln IV oder zu Enantiomeren der Formeln IVa oder IVb 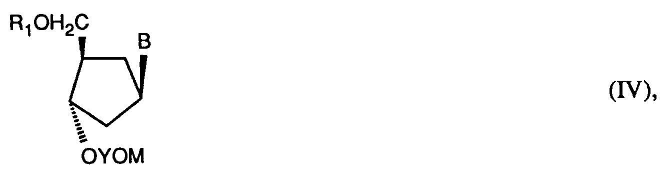 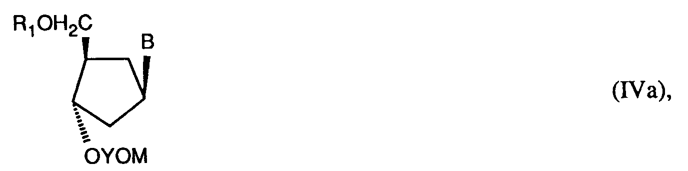 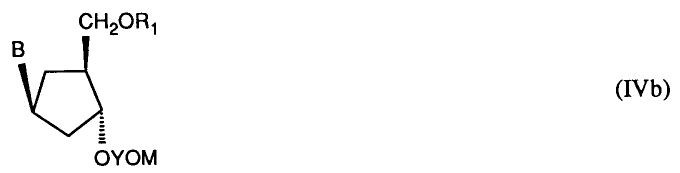 umsetzt, worin M für H, ein Alkalimetall oder eine nicht-nukleophile Base steht und Y die zuvor angegebene Bedeutung hat, und
c) die Verbindungen der Formeln IV, IVa und IVb durch Abspaltung der Schutzgruppe R₁ und der Gruppe YOM in die Verbindungen der Formeln I, Ia und Ib umwandelt.

2. Verfahren gemäss Anspruch 1, worin R₁ lineares oder verzweigtes C₁-C₈-Alkyl, C₇-C₁₂-Aralkyl, Triphenylsilyl, Alkyldiphenylsilyl, Dialkylphenylsilyl und Trialkylsilyl mit 1 bis 20 C-Atomen in den Alkylgruppen, C₂-C₁₂-Acyl, R₁₇-SO₂-, worin R₁₇ C₁-C₁₂-Alkyl, C₅- oder C₆-Cycloalkyl, Phenyl, Benzyl, C₁-C₁₂-Alkylphenyl, C₁-C₁₂-Alkylbenzyl, oder Halogenphenyl oder Halogenbenzyl bedeutet, oder C₁-C₁₂-Alkoxycarbonyl, Phenyloxycarbonyl, Benzyloxycarbonyl, Methyl- oder Methoxy- oder Chlorphenyloxycarbonyl oder -benzyloxycarbonyl darstellt.

3. Verfahren gemäss Anspruch 2, worin R₁ lineares oder verzweigtes C₁-C₄-Alkyl, C₇-C₁₂-Alkyl, Trialkylsilyl mit 1 bis 12 C-Atomen in den Alkylgruppen, C₂-C₈-Acyl, R₁₇-SO₂-, worin R₁₇ C₁-C₆-Alkyl, Phenyl, Benzyl, C₁-C₄-Alkylphenyl, C₁-C₄-Alkylbenzyl, oder Halogenphenyl oder Halogenbenzyl bedeutet, oder R₁ C₁-C₈-Alkoxycarbonyl, Phenoxycarbonyl oder Benzyloxycarbonyl darstellt.

4. Verfahren gemäss Anspruch 1, worin R₁ Methyl, Ethyl, n- und i-Propyl, n-, i- und t-Butyl; Benzyl, Methylbenzyl, Dimethylbenzyl, Methoxybenzyl, Dimethoxybenzyl, Brombenzyl; Diphenylmethyl, Di(methylphenyl)methyl, Di(dimethylphenyl)methyl, Di(methoxyphenyl)methyl, Di(dimethoxyphenyl)methyl, Trityl, Tri(methylphenyl)methyl, Tri(dimethylphenyl)methyl, Tri(methoxyphenyl)methyl, Tri(dimethoxyphenyl)methyl, Monomethoxytrityl, Dimethoxytrityl; Pixyl; Trimethylsilyl, Triethylsilyl, Tri-n-propylsilyl, i-Propyl-dimethylsilyl, t-Butyl-dimethylsilyl, t-Butyl-diphenylsilyl, n-Octyl-dimethylsilyl, (1,1,2,2-Tetramethylethyl)-dimethylsilyl; Acetyl, Propanoyl, Butanoyl, Pentanoyl, Hexanoyl, Benzoyl, Methylbenzoyl, Methoxybenzoyl, Chlorbenzoyl und Brombenzoyl; Methyl-, Ethyl-, Propyl-, Butyl-, Phenyl-, Benzyl-, p-Brom-, p-Methoxy- und p-Methylphenylsulfonyl; Methoxy-, Ethoxy-, n- oder i-Propoxy- oder n-, i- oder t-Butoxycarbonyl, oder Phenyloxycarbonyl, Benzyloxycarbonyl, Methyl- oder Methoxy- oder Chlorphenyloxycarbonyl oder -benzyloxycarbonyl darstellt.

5. Verfahren gemäss Anspruch 1, worin es sich bei B als Purinrest oder einem analogen Rest davon um Reste der Formeln V, Va, Vb, Vc, Vd oder Ve handelt, 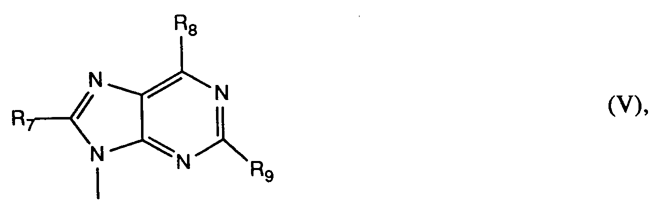 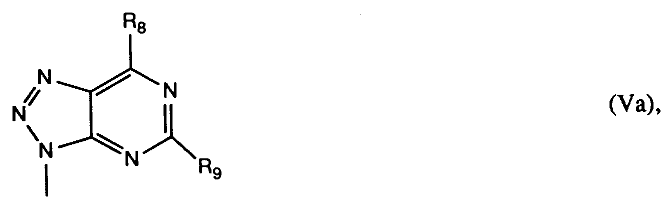 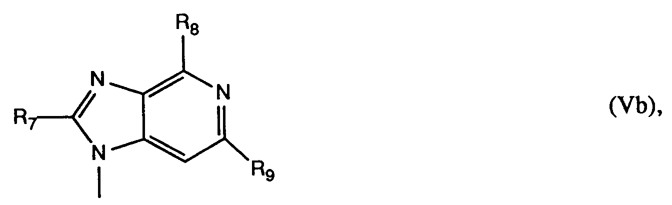 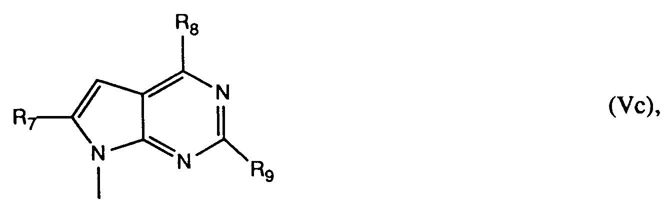 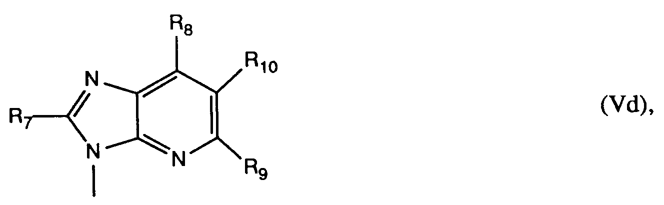 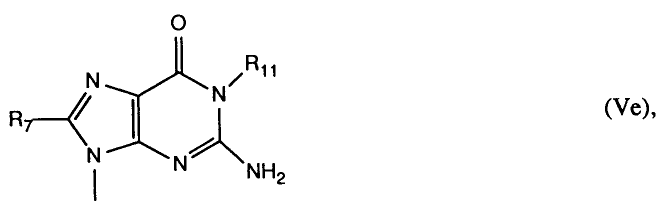 worin R₇ für H, Cl, Br, NH₂ oder OH steht, und R₈, R₉ und R₁₀ unabhängig voneinander H, OH, SH, NH₂, NHNH₂, NHOH, NHOAlkyl mit 1 bis 12 C-Atomen, F, Cl, Br, Alkyl oder Hydroxyalkyl oder Aminoalkyl oder Alkoxy oder Alkylthio mit 1 bis 12 C-Atomen, wobei die Hydroxyl- und Aminogruppen unsubstituiert oder mit einer Schutzgruppe substituiert sind, Phenyl, Benzyl, Primäramino mit 1 bis 20 C-Atomen oder Sekundäramino mit 2 bis 30 C-Atomen bedeuten, und R₁₁ H oder C₁-C₄-Alkyl darstellt.

6. Verfahren gemäss Anspruch 5, dadurch gekennzeichnet dass, in den Formeln V bis Ve die Schutzgruppe für Hydroxyl- und Aminogruppen C₁-C₈-Acyl darstellt.

7. Verfahren gemäss Anspruch 5, dadurch gekennzeichnet, dass Primäramino 1 bis 12 C-Atome und das Sekundäramino 2 bis 12 C-Atome enthält.

8. Verfahren gemäss Anspruch 5, dadurch gekennzeichnet, dass es sich bei dem Primäramino und Sekundäramino um Reste der Formel R₁₄R₁₅N handelt, worin R₁₄ für H steht oder unabhängig die Bedeutung von R₁₅ hat, und R₁₅ C₁-C₂₀-Alkyl, -Aminoalkyl, -Hydroxyalkyl; Carboxyalkyl oder Carbalkoxyalkyl, wobei die Carbalkoxygruppe 2 bis 8 C-Atome enthält und die Alkylgruppe 1 bis 6, bevorzugt 1 bis 4 C-Atome; C₂-C₂₀-Alkenyl; Phenyl, Mono- oder Di-(C₁-C₄-Alkyl- oder Alkoxy)phenyl, Benzyl, Mono- oder Di-(C₁-C₄-Alkyl- oder -Alkoxy)benzyl; oder 1,2-, 1,3- oder 1,4-Imidazolyl-C₁-C₆-Alkyl darstellt, oder R₁₄ und R₁₅ zusammen Tetra- oder Pentamethylen, 3-Oxa-1,5-pentylen, -CH₂-NR₁₆-CH₂CH₂- oder -CH₂CH₂-NR₁₆-CH₂CH₂- darstellen, worin R₁₆ für H oder -C₁-C₄-Alkyl steht, wobei die Aminogruppe im Aminoalkyl unsubstituiert oder mit ein oder zwei C₁-C₄-Alkyl oder -Hydroxyalkylgruppen substituiert ist und die Hydroxylgruppe im Hydroxyalkyl gegebenenfalls mit C₁-C₄-Alkyl verethert ist.

9. Verfahren gemäss Anspruch 8, dadurch gekennzeichnet, dass es sich bei dem Primäramino und Sekundäramino um Methyl-, Ethyl-, Dimethyl-, Diethyl-, Allyl-, Mono- oder Di-(hydroxy-eth-2-yl)-, Phenyl- und Benzyl-, Acetyl- und Benzoylamino handelt.

10. Verfahren gemäss Anspruch 5, dadurch gekennzeichnet, dass R₇ in den Formeln V, Vb, Vc, Vd und Ve für Wasserstoff steht.

11. Verfahren gemäss Anspruch 5, dadurch gekennzeichnet, dass R₁₀ in Formel Vd für Wasserstoff steht.

12. Verfahren gemäss Anspruch 5, dadurch gekennzeichnet, dass R₈ und R₉ in den Formeln V, Va, Vb, Vc, Vd und Ve unabhängig voneinander H, F, Cl, Br, OH, SH, NH₂, NHOH, NHNH₂, Methylamino, Dimethylamino, Benzoylamino, Methoxy, Ethoxy und Methylthio darstellen.

13. Verfahren gemäss Anspruch 1, worin in den Formeln I, Ia und Ib B als Purinrest oder als ein Rest eines Purinanalogen aus der Reihe Adenin, N-Methyladenin, N-Benzoyladenin, 2-Methylthioadenin, 2-Aminoadenin, 6-Hydroxypurin, 2-Amino-6-chlorpurin, 2-Amino-6-methylthiopurin, Guanin, N-Isobutyrylguanin ausgewählt ist.

14. Verfahren gemäss Anspruch 1, worin es sich bei B in Formel I als analogem Pyrimidinrest um Uracil-, Thymin- und Cytosinreste der Formeln VI, VIa und VIb handelt, 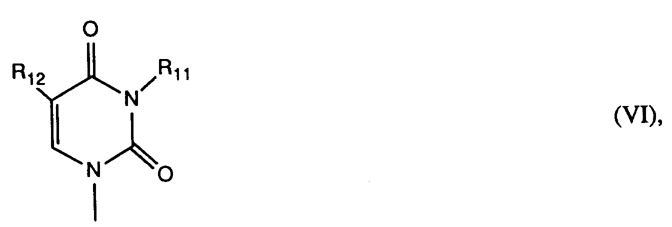 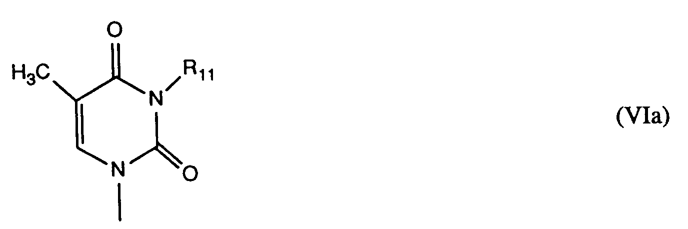 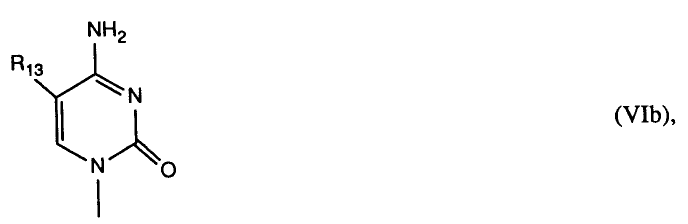 worin R₁₁ H oder C₁-C₄-Alkyl bedeutet, und R₁₂ und R₁₃ unabhängig voneinender die in Anspruch 5 für R₈ angegebene Bedeutung haben, und die Wasserstoffatome der NH₂-Gruppe in Formel VIb mit C₁-C₆-Alkyl oder Benzoyl substituiert sein können, sowie die Dihydroderivate der Reste der Formeln VI, VIa und VIb.

15. Verfahren gemäss Anspruch 14, dadurch gekennzeichnet, dass R₁₁ in den Formeln VI und VIa für H oder Methyl steht.

16. Verfahren gemäss Anspruch 14, dadurch gekennzeichnet, dass R₁₂ in Formel VI H, C₁-C₆-Alkyl oder -Hydroxyalkyl, F, Cl, Br, NH₂, Benzoylamino, Mono- oder Di-C₁-C₆-alkylamino darstellt.

17. Verfahren gemäss Anspruch 14, dadurch gekennzeichnet, dass R₁₃ in Formel VIb H, C₁-C₆-Alkyl oder -Alkoxy oder -Hydroxyalkyl, F, Cl, Br, NH₂, Benzoylamino, Mono- oder Di-C₁-C₆-alkylamino darstellt.

18. Verfahren gemäss Anspruch 16, dadurch gekennzeichnet, dass R₁₂ H, F, Cl, Br, NH₂, NHCH₃, N(CH₃)₂ oder C₁-C₄-Alkyl bedeutet.

19. Verfahren gemäss Anspruch 17, dadurch gekennzeichnet, dass R₁₃ H, C₁-C₄-Alkyl, NH₂, NHCH₃ oder (CH₃)₂N dar bedeutet.

20. Verfahren gemäss Anspruch 1, dadurch gekennzeichnet, dass sich die Reste B in den Formeln I, Ia und Ib als Pyrimidinanaloge von Uracil, Thymin, Cytosin, 5-Fluoruracil, 5-Chloruracil, 5-Bromuracil, Dihydrouracil, nd 5-Methylcytosin ableiten.

21. Verfahren gemäss Anspruch 1, dadurch gekennzeichnet, dass R₂ C₁-C₁₂--Alkyl, C₅-oder C₆-Cycloalkyl, Phenyl, Benzyl, oder mit C₁-C₄-Alkyl oder C₁-C₄-Alkoxy substituiertes Phenyl oder Benzyl darstellt.

22. Verfahren gemäss Anspruch 1, dadurch gekennzeichnet, dass R₃ und R₄ H, C₁-C₁₂-Alkyl, C₅- oder C₆-Cycloalkyl, Phenyl, Benzyl, oder mit C₁-C₄-Alkyl oder C₁-C₄-Alkoxy substituiertes Phenyl oder Benzyl bedeuten.

23. Verfahren gemäss Anspruch 1, dadurch gekennzeichnet, dass R₅ C₁-C₆-Alkyl, C₅- oder C₆-Cycloalkyl, Phenyl, Benzyl, oder mit C₁-C₄-Alkyl oder C₁-C₄-Alkoxy substituiertes Phenyl oder Benzyl darstellt.

24. Verfahren gemäss Anspruch 1, dadurch gekennzeichnet, dass R₆ C₁-C₆-Alkyl oder -Alkoxy, C₅- oder C₆-Cycloalkyl oder -Cycloalkoxy, Phenyl oder Phenyloxy, Benzyl oder Benzyloxy, oder mit C₁-C₄-Alkyl oder C₁-C₄-Alkoxy substituiertes Phenyl, Phenyloxy, Benzyl oder Benzyloxy darstellt.

25. Verfahren gemäss Anspruch 1, dadurch gekennzeichnet, dass es sich bei der Abgangsgruppe X um Halogenid, C₁-C₆-Alkoxy, Phenoxy, Benzyloxy oder um offenkettiges oder cyclisches Sekundäramino mit 2 bis 12 C-Atomen handelt.

26. Verfahren gemäss Anspruch 1, dadurch gekennzeichnet, dass die Verfahrensstufen a) und b) in einem inerten Lösungsmittel durchgeführt werden.

27. Verfahren gemäss Anspruch 1, dadurch gekennzeichnet, dass in der Verfahrensstufe a) die Umsetzung mit einer Verbindung der Formel X-CO-X vorgenommen wird, worin X für Imidazolyl steht, oder mit einer Verbindung der Formel X-SO-X durchgeführt wird, worin X für Cl oder Br steht, und die erhaltene cyclische Sulfoxidverbindung anschliessend zur cyclischen Sulfodioxidverbindung oxidiert wird.

28. Verfahren gemäss Anspruch 1, dadurch gekennzeichnet, dass die Reaktionstemperatur in der Verfahrensstufe a) -20 bis 200 °C beträgt.

29. Verfahren gemäss Anspruch 28, dadurch gekennzeichnet, dass die Reaktionstemperatur in der Verfahrensstufe a) 0 bis 120 °C beträgt.

30. Verfahren gemäss Anspruch 1, dadurch gekennzeichnet, dass man in der Verfahrensstufe a) säurebindende Mittel mitverwendet, wenn bei der Reaktion Säuren HX gebildet werden.

31. Verfahren gemäss Anspruch 1, dadurch gekennzeichnet, dass man als säurebindende Mittel tertiäre Amine verwendet.

32. Verfahren gemäss Anspruch 1, dadurch gekennzeichnet, dass man das säurebindende Mittel im Überschuss verwendet.

33. Verfahren gemäss Anspruch 1, dadurch gekennzeichnet, dass es sich bei der Abgangsgruppe X um Chlorid, Bromid, Metoxy, Ethoxy oder Imidazolyl handelt.

34. Verfahren gemäss Anspruch 1, dadurch gekennzeichnet, dass man in der Verfahrensstufe a) einen Überschuss des zur Einführung der Gruppe Y verwendeten Reaktanden einsetzt.

35. Verfahren gemäss Anspruch 1, dadurch gekennzeichnet, dass man in der Verfahrensstufe b) eine nicht-nukleophile Base mitverwendet.

36. Verfahren gemäss Anspruch 35, dadurch gekennzeichnet, dass es sich bei der nichtnukleophilen Base um 1,8-Diaza-bicyclo-[5,4,0]-undec-7-en handelt.

37. Verfahren gemäss Anspruch 1, dadurch gekennzeichnet, dass die Reaktionstemperatur in der Verfahrensstufe b) -20 bis 200 °C beträgt.

38. Verfahren gemäss Anspruch 37, dadurch gekennzeichnet, dass die Reaktionstemperatur in der Verfahrensstufe b) 0 bis 150 °C beträgt.

39. Verfahren gemäss Anspruch 1, dadurch gekennzeichnet, dass man in der Verfahrensstufe c) die Schutzgruppe R₁ und die Gruppe YOM gleichzeitig oder nacheinander abspaltet.

40. Verfahren gemäss Anspruch 1, dadurch gekennzeichnet, dass man in der Verfahrensstufe c) die Gruppe YOM hydrolytisch abspaltet.

41. Verfahren gemäss Anspruch 40, dadurch gekennzeichnet, dass man die Gruppe YOM mit verdünnten wässrigen Mineralsäuren in alkoholischer Lösung abspaltet.

42. Verbindungen der Formeln II, IIa und IIb , 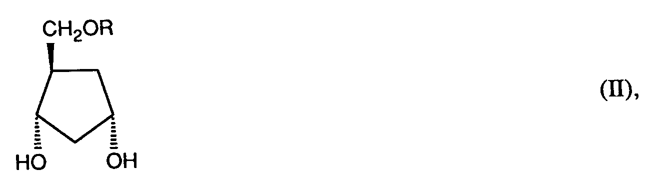 oder der Enantiomeren der Formeln (Ia) und (Ib) 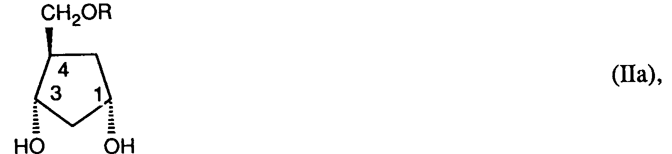 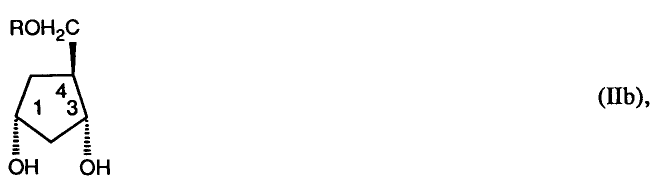 worin B für den Rest einer Nukleinbase aus der Reihe Purin oder einem Purinanalogen oder Pyrimidinanalogen steht und R H oder eine Schutzgruppe R₁ bedeutet.

43. Verbindungen gemäss Anspruch 42, dadurch gekennzeichnet, dass R₁ lineares oder verzweigtes C₁-C₈-Alkyl, C₇-C₁₂-Aralkyl, Triphenylsilyl, Alkyldiphenylsilyl, Dialkylphenylsilyl und Trialkylsilyl mit 1 bis 20 C-Atomen in den Alkylgruppen, C₂-C₁₂-Acyl, R₁₇-SO₂-, worin R₁₇ C₁-C₁₂-Alkyl, C₅- oder C₆-Cycloalkyl, Phenyl, Benzyl, C₁-C₁₂-Alkylphenyl, C₁-C₁₂-Alkylbenzyl, oder Halogenphenyl oder Halogenbenzyl bedeutet, oder C₁-C₁₂-Alkoxycarbonyl, Phenyloxycarbonyl, Benzyloxycarbonyl, Methyl- oder Methoxy- oder Chlorphenyloxycarbonyl oder -benzyloxycarbonyl darstellt.

44. Verbindungen gemäss Anspruch 42, worin R₁ lineares oder verzweigtes C₁-C₄-Alkyl, C₇-C₁₂-Alkyl, Trialkylsilyl mit 1 bis 12 C-Atomen in den Alkylgruppen, C₂-C₈-Acyl, R₁₇-SO₂-, worin R₁₇ C₁-C₆-Alkyl, Phenyl, Benzyl, C₁-C₄-Alkylphenyl, C₁-C₄-Alkylbenzyl, oder Halogenphenyl oder Halogenbenzyl bedeutet, oder R₁ C₁-C₈-Alkoxycarbonyl, Phenoxycarbonyl oder Benzyloxycarbonyl darstellt.

45. Verbindungen gemäss Anspruch 42, worin R₁ Methyl, Ethyl, n- und i-Propyl, n-, i- und t-Butyl; Benzyl, Methylbenzyl, Dimethylbenzyl, Methoxybenzyl, Dimethoxybenzyl, Brombenzyl; Diphenylmethyl, Di(methylphenyl)methyl, Di(dimethylphenyl)methyl, Di(methoxyphenyl)methyl, Di(dimethoxyphenyl)methyl, Trityl, Tri(methylphenyl)methyl, Tri(dimethylphenyl)methyl, Tri(methoxyphenyl)methyl, Tri(dimethoxyphenyl)methyl, Monometoxytrityl, Dimethoxytrityl; Pixyl; Trimethylsilyl, Triethylsilyl, Tri-n-propylsilyl, i-Propyl-dimethylsilyl, t-Butyl-dimethylsilyl, t-Butyl-diphenylsilyl, n-Octyl-dimethylsilyl, (1,1,2,2-Tetramethylethyl)-dimethylsilyl; Acetyl, Propanoyl, Butanoyl, Pentanoyl, Hexanoyl, Benzoyl, Methylbenzoyl, Methoxybenzoyl, Chlorbenzoyl und Brombenzoyl; Methyl-, Ethyl-, Propyl-, Butyl-, Phenyl-, Benzyl-, p-Brom-, p-Methoxy- und p-Methylphenylsulfonyl; Methoxy-, Ethoxy-, n- oder i-Propoxy- oder n-, i- oder t-Butoxycarbonyl, oder Phenyloxycarbonyl, Benzyloxycarbonyl, Methyl- oder Methoxy- oder Chlorphenyloxycarbonyl oder -benzyloxycarbonyl darstellt.

46. Verbindungen der Formeln III, IIIa und IIIb 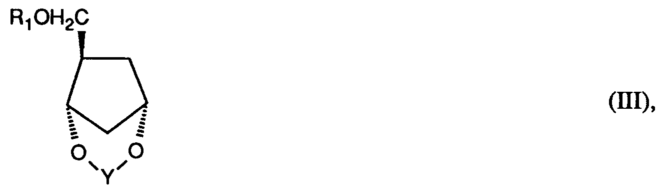 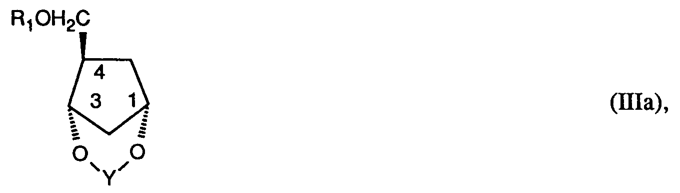 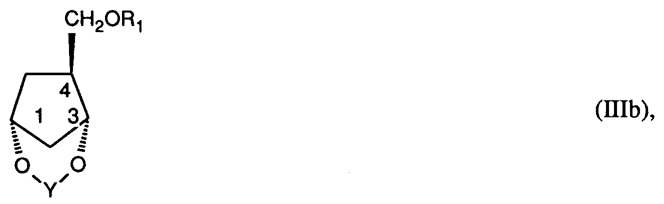 worin Y die Gruppen -C(O)-, -C(S)-, -SO-, -SO₂-, R₂NH-CH=, R₃R₄N-CR₅=, R₅P= und R₆(O)P= darstellt, und R₁ eine Schutzgruppe und R₂ bis R₆ die in Anspruch 1 angegebenen Bedeutungen haben.

47. Verbindungen gemäss Anspruch 46, dadurch gekennzeichnet, dass Y für -CO-, -SO- oder -SO₂- steht.

48. Verbindungen gemäss Anspruch 46, dadurch gekennzeichnet, dass R₁ lineares oder verzweigtes C₁-C₈-Alkyl, C₇-C₁₂-Aralkyl, Triphenylsilyl, Alkyldiphenylsilyl, Dialkylphenylsilyl und Trialkylsilyl mit 1 bis 20 C-Atomen in den Alkylgruppen, C₂-C₁₂-Acyl, R₁₇-SO₂-, worin R₁₇ C₁-C₁₂-Alkyl, C₅- oder C₆-Cycloalkyl, Phenyl, Benzyl, C₁-C₁₂-Alkylphenyl, C₁-C₁₂-Alkylbenzyl, oder Halogenphenyl oder Halogenbenzyl bedeutet, oder C₁-C₁₂-Alkoxycarbonyl, Phenyloxycarbonyl, Benzyloxycarbonyl, Methyl- oder Methoxy- oder Chlorphenyloxycarbonyl oder -benzyloxycarbonyl darstellt.

49. Verbindungen gemäss Anspruch 46, worin R₁ lineares oder verzweigtes C₁-C₄-Alkyl, C₇-C₁₂-Aralkyl, Trialkylsilyl mit 1 bis 12 C-Atomen in den Alkylgruppen, C₂-C₈-Acyl, R₁₇-SO₂-, worin R₁₇ C₁-C₆-Alkyl, Phenyl, Benzyl, C₁-C₄-Alkylphenyl, C₁-C₄-Alkylbenzyl, oder Halogenphenyl oder Halogenbenzyl bedeutet, oder R₁ C₁-C₈-Alkoxycarbonyl, Phenoxycarbonyl oder Benzyloxycarbonyl darstellt.

50. Verbindungen gemäss Anspruch 46, worin R₁ Methyl, Ethyl, n- und i-Propyl, n-, i- und t-Butyl; Benzyl, Methylbenzyl, Dimethylbenzyl, Methoxybenzyl, Dimethoxybenzyl, Brombenzyl; Diphenylmethyl, Di(methylphenyl)methyl, Di(dimethylphenyl)methyl, Di(methoxyphenyl)methyl, Di(dimethoxyphenyl)methyl, Trityl, Tri(methylphenyl)methyl, Tri(dimethylphenyl)methyl, Tri(methoxyphenyl)methyl, Tri(dimethoxyphenyl)methyl, Monomethoxytrityl, Dimethoxytrityl; Pixyl; Trimethylsilyl, Triethylsilyl, Tri-n-propylsilyl, i-Propyl-dimethylsilyl, t-Butyl-dimethylsilyl, t-Butyl-diphenylsilyl, n-Octyl-dimethylsilyl, (1,1,2,2-Tetramethylethyl)-dimethylsilyl; Acetyl, Propanoyl, Butanoyl, Pentanoyl, Hexanoyl, Benzoyl, Methylbenzoyl, Methoxybenzoyl, Chlorbenzoyl und Brombenzoyl; Methyl-, Ethyl-, Propyl-, Butyl-, Phenyl-, Benzyl-, p-Brom-, p-Methoxy- und p-Methylphenylsulfonyl; Methoxy-, Ethoxy-, n- oder i-Propoxy- oder n-, i- oder t-Butoxycarbonyl, oder Phenyloxycarbonyl, Benzyloxycarbonyl, Methyl- oder Methoxy- oder Chlorphenyloxycarbonyl oder -benzyloxycarbonyl darstellt.
